(19) Europäisches Patentamt
European Patent Office
Office européen des brevets

(11) EP 4 692 062 A1

(12) **EUROPEAN PATENT APPLICATION**
published in accordance with Art. 153(4) EPC

(43) Date of publication:
**11.02.2026 Bulletin 2026/07**

(21) Application number: **24781293.6**

(22) Date of filing: **29.03.2024**

(51) International Patent Classification (IPC):
***C07D 211/96*** (2006.01) ***C07C 311/21*** (2006.01)
***C07C 311/19*** (2006.01) ***C07D 207/48*** (2006.01)
***A61K 8/46*** (2006.01) ***A61K 8/49*** (2006.01)
***A61K 31/445*** (2006.01) ***A61K 31/40*** (2006.01)
***A61P 17/00*** (2006.01)

(52) Cooperative Patent Classification (CPC):
**A61K 8/46; A61K 8/49; A61K 31/40; A61K 31/445; A61P 17/00; C07C 311/19; C07C 311/21; C07D 207/48; C07D 211/96**

(86) International application number:
**PCT/KR2024/004030**

(87) International publication number:
**WO 2024/205303 (03.10.2024 Gazette 2024/40)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **30.03.2023 KR 20230042219**
**27.03.2024 KR 20240041934**

(71) Applicant: **Amorepacific Corporation**
**Seoul 04386 (KR)**

(72) Inventors:
- **JANG, Geunhyuk**
  **Yongin-si, Gyeonggi-do 17074 (KR)**
- **YOU, Jaewon**
  **Yongin-si, Gyeonggi-do 17074 (KR)**
- **LEE, Jinyoung**
  **Yongin-si, Gyeonggi-do 17074 (KR)**
- **NAM, Jin**
  **Yongin-si, Gyeonggi-do 17074 (KR)**
- **PARK, Wonseok**
  **Yongin-si, Gyeonggi-do 17074 (KR)**
- **BAEK, Heungsoo**
  **Yongin-si, Gyeonggi-do 17074 (KR)**

(74) Representative: **Schön, Christoph**
**Dr. Schön, Neymeyr & Partner mbB**
**Bavariaring 26**
**80336 München (DE)**

(54) **NOVEL AMIDE-BASED COMPOUND AND COMPOSITION COMPRISING SAME**

(57) The present specification relates to a novel amide-based compound derived from an amino acid structure such as valine, leucine, phenylalanine, proline, or pipecolic acid and having MMP-1, MMP-2, or MMP-9 inhibitory activities even in a tiny amount, an isomer thereof, a pharmaceutically acceptable salt thereof, a hydrate thereof, or a solvate thereof, and a composition comprising same.

EP 4 692 062 A1

## Description

### [Technical Field]

The present application claims priority to Korean Patent Application Nos. 10-2023-0042219 filed on March 30, 2023 and 10-2024-0041934 filed on March 27, 2024, and the entire contents of which is incorporated herein for all purposes by this reference.

Disclosed herein are a novel amide-based compound and a composition comprising the same.

### [Background Art]

An amino acid is an organic compound that has an amino group, a carboxyl group, and a specific side chain (R group). The elements present in all amino acids are carbon (C), hydrogen (H), oxygen (O), and nitrogen (N), while sulfur (S) is present in the side chains of cysteine and methionine, and selenium (Se) is present in the side chain of selenocysteine, which is a less common amino acid. In particular, among the 21 amino acids commonly found in all living organisms, nine amino acids that humans cannot synthesize are called essential amino acids, namely, phenylalanine, valine, threonine, tryptophan, methionine, leucine, isoleucine, lysine, and histidine.

Meanwhile, in order to secure fundamental technologies for the promotion of the domestic cosmetics industry, there has been a continuous need for novel compounds. Therefore, to develop new materials for functional cosmetics, novel compounds derived from well-known amino acid compounds such as valine, leucine, phenylalanine, proline, and pipecolic acid (a proline analog) have been synthesized.

### [Disclosure]

[Technical Problem]

In an aspect, the present disclosure is to provide a novel compound derived from an amino acid structure such as valine, leucine, phenylalanine, proline or pipecolic acid, and a composition comprising the same.

In another aspect, the present disclosure is to provide a novel compound having an inhibitory efficacy on Matrix MetalloProteinases (MMPs) and a composition comprising the same.

[Technical Solution]

In an aspect, the present disclosure provides a compound represented by the following formula 1, an isomer thereof, a pharmaceutically acceptable salt thereof, a hydrate thereof, or a solvate thereof:

[Formula 1]

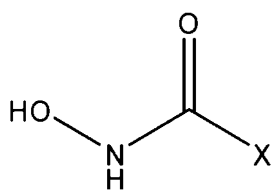

In Formula 1,
X is represented by any one of the following formulas 1-1 to 1-5,

[Formula 1-1]

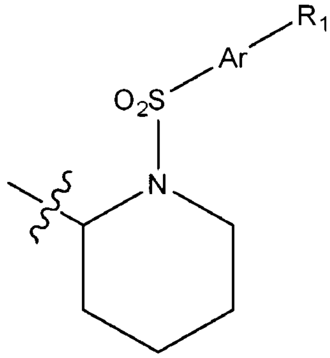

[Formula 1-2]

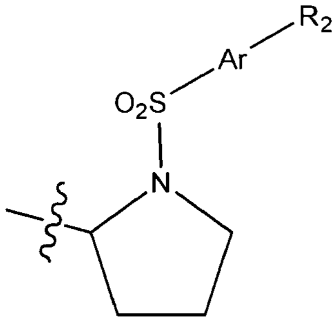

[Formula 1-3]

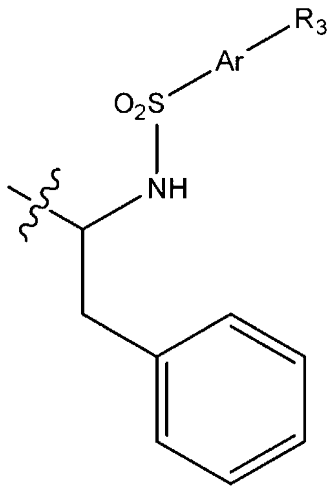

[Formula 1-4]

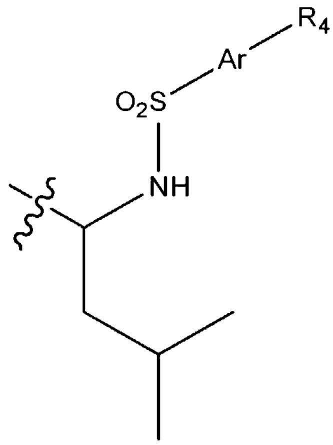

[Formula 1-5]

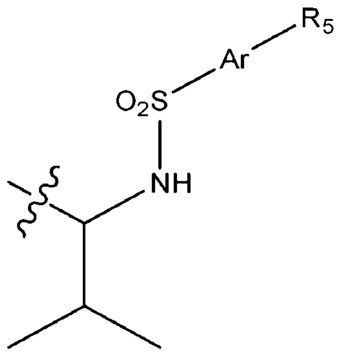

In Formula 1-1,

Ar is a phenyl unsubstituted or substituted with 1 to 3 $R_1$,
$R_1$ is a hydrogen, a phenyl group, a $C_1$-$C_4$ alkoxy group, a $C_1$-$C_3$ alkyl group, a fluoro group, an adamantane group, an acetylamino group, or a hydroxyiminoethyl group, when there are 2 or 3 $R_1$ residues, these $R_1$ residues are identical with each other,
In Formula 1-2,
Ar is a phenyl substituted with one $R_2$,
$R_2$ is a $C_1$-$C_4$ alkyl group, a $C_1$-$C_4$ alkoxy group, an adamantane group, a acetyl group, or a acetylamino group,
In Formula 1-3,
Ar is a phenyl substituted with 1-3 $R_3$ or an unsubstituted naphthalene,
$R_3$ is a hydrogen or a $C_1$-$C_4$ alkyl group, when there are 2 or 3 $R_3$ residues, these $R_3$ residues are identical with each other, preferably $R_3$ is a $C_1$-$C_4$ alkyl group,
In Formula 1-4,
Ar is a phenyl unsubstituted or substituted with 1 to 3 $R_4$,
$R_4$ is a hydrogen, a phenyl group, a $C_1$-$C_4$ alkyl group, or a $C_1$-$C_4$ alkoxy group, when there are 2 or 3 $R_4$ residues, these $R_4$ residues are identical with each other,
In Formula 1-5,
Ar is a phenyl substituted with 1 to 3 $R_5$,
$R_5$ is a $C_1$-$C_4$ alkyl group, when there are 2 or 3 $R_5$ residues, these $R_5$ residues are identical with each other.

In another aspect, the present disclosure provides a composition comprising the compound represented by formula 1 as an active ingredient.

In another aspect, the present disclosure provides a method for preparing the compound represented by formula 1, comprising reacting an amino acid selected from pipecolinic acid, phenylalanine, leucine, valine and proline with any one selected from sulfonic acid, sulfonyl halide, sulfanilic acid, and sulfanilyl halide.

[Advantageous Effects]

In an aspect, the novel compound and the composition comprising the same as disclosed herein can exhibit an inhibitory efficacy on various MMPs at low nanomolar (nM) concentrations, for example at a concentration of 500 nM or lower.

**[Mode for Invention]**

Hereinafter, the present disclosure will be described in more detail through the following embodiments. However, the following embodiments are intended to provide only for illustrative purpose to aid understanding of the present disclosure, without limiting the scope of the present disclosure thereto.

Exemplary embodiments of the present disclosure provide a compound represented by the following formula 1, an isomer thereof, a pharmaceutically acceptable salt thereof, a hydrate thereof, or a solvate thereof:

[Formula 1]

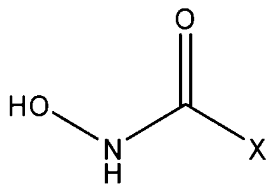

In Formula 1,

X is represented by any one of the following formulas 1-1 to 1-5,

[Formula 1-1]

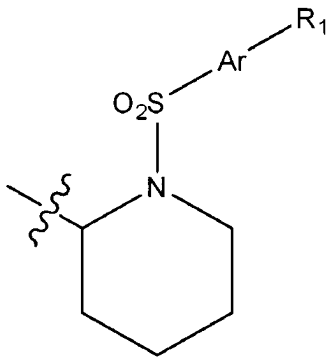

[Formula 1-2]

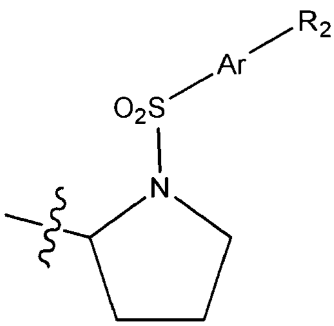

[Formula 1-3]

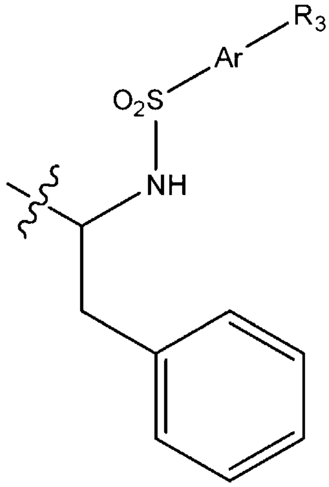

[Formula 1-4]

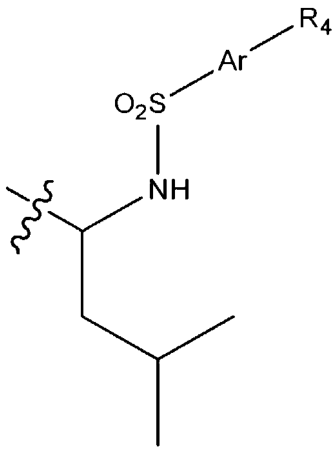

[Formula 1-5]

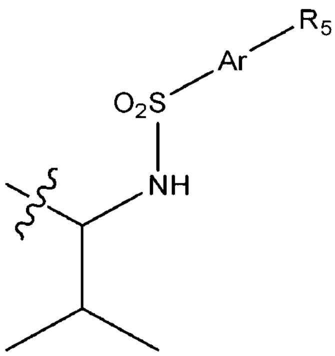

In Formula 1-1,

Ar is a phenyl unsubstituted or substituted with 1 to 3 $R_1$,
$R_1$ is a hydrogen, a phenyl group, a $C_1$-$C_4$ alkoxy group, a $C_1$-$C_3$ alkyl group, a fluoro group, an adamantane group, an acetylamino group, or a hydroxyiminoethyl group, when there are 2 or 3 $R_1$ residues, these $R_1$ residues are identical with each other,
In Formula 1-2,
Ar is a phenyl substituted with one $R_2$,
$R_2$ is a $C_1$-$C_4$ alkyl group, a $C_1$-$C_4$ alkoxy group, an adamantane group, a acetyl group, or a acetylamino group,

In Formula 1-3,
Ar is a phenyl substituted with 1-3 $R_3$ or an unsubstituted naphthalene,
$R_3$ is a hydrogen or a $C_1$-$C_4$ alkyl group, when there are 2 or 3 $R_3$ residues, these $R_3$ residues are identical with each other, preferably $R_3$ is a $C_1$-$C_4$ alkyl group,
In Formula 1-4,
Ar is a phenyl unsubstituted or substituted with 1 to 3 $R_4$,
$R_4$ is a hydrogen, a phenyl group, a $C_1$-$C_4$ alkyl group, or a $C_1$-$C_4$ alkoxy group, when there are 2 or 3 $R_4$ residues, these $R_4$ residues are identical with each other,
In Formula 1-5,
Ar is a phenyl substituted with 1 to 3 $R_5$,
$R_5$ is a $C_1$-$C_4$ alkyl group, when there are 2 or 3 $R_5$ residues, these $R_5$ residues are identical with each other.

For example, in Formulas 1-1 to 1-5, $R_1$ to $R_5$ may be substituted at the para position of Ar, respectively.

In this specification, the alkyl group may be straight chain or branched, for example, methyl, ethyl, propyl, n-propyl, isopropyl, butyl, n-butyl, isobutyl, tert-butyl, or sec-butyl.

In this specification, the alkoxy group may be straight chain, branched chain, or ring chain, for example, methoxy, ethoxy, n-propoxy, isopropoxy, i-propyloxy, n-butoxy, isobutoxy, tert-butoxy, or sec-butoxy.

As used herein, the term "isomer" includes, in particular, an optical isomer (e.g., essentially pure enantiomer, essentially pure diastereomer or a mixture thereof), as well as a conformational isomer (i.e., an isomer that differs only in the angle of one or more chemical bonds), a positional isomer (especially, a tautomer), or a geometric isomer (e.g., a cis-trans isomer).

As used herein, the term "essentially pure" means that, for example, when used in relation to an enantiomer or a diastereomer, the concrete compound including the enantiomer or the diastereomer is present at about 90 % or more, preferably about 95 % or more, more preferably about 97 % or more or about 98 % or more, even more preferably about 99 % or more, and even more preferably about 99.5 % or more (w/w).

As used herein, the term "pharmaceutically acceptable" means that it has been or is expected to be approved by a government or an equivalent regulatory authority that the compound is available to an animal, more specifically a human being, by avoiding a significant toxic effect when used in a normal medicinal dosage, or means that it listed in a pharmacopoeia or otherwise recognized by other general pharmacopoeias.

As used herein, the term "pharmaceutically acceptable salt" refers to a salt according to an aspect of the present disclosure that is pharmaceutically acceptable and has the desired pharmacological activity of a parent compound. The salt may include (1) an acid addition salt formed with the inorganic acid such as a hydrochloric acid, a hydrobromic acid, a sulfuric acid, a nitric acid, and a phosphoric acid; or an acid addition salt formed with the organic acid such as an acetic acid, a propionic acid, a hexanoic acid, a cyclopentanepropionic acid, a glycolic acid, a pyruvic acid, a lactic acid, a malonic acid, a succinic acid, a malic acid, a maleic acid, a fumaric acid, a tartaric acid, a citric acid, a benzoic acid, 3-(4-hydroxybenzoyl) benzoic acid, a cinnamic acid, a mandelic acid, a methanesulfonic acid, an ethanesulfonic acid, 1,2-ethane-disulfonic acid, 2-hydroxyethanesulfonic acid, a benzenesulfonic acid, 4-chlorobenzenesulfonic acid, 2-naphthalenesulfonic acid, 4-Toluenesulfonic acid, a camphorsulfonic acid, 4-methylbicyclo[2,2,2]-oct-2-ene-1-carboxylic acid, a glucoheptonic acid, 3-phenylpropionic acid, a trimethylacetic acid, a tert-butylacetic acid, a lauryl sulfate, a gluconic acid, a glutamic acid, a hydroxynaphthoic acid, a salicylic acid, a stearic acid, and a muconic acid; or (2) a salt formed when an acidic proton present in the parent compound is substituted.

As used herein, the term "hydrate" refers to a compound to which water is bound, and is a broad concept that includes an embedded compound having no chemical bond between the water and the compound.

As used herein, the term "solvate" refers to a higher-order compound formed between solute molecules or ions and solvent molecules or ions.

According to an embodiment of the present disclosure, the compound represented by formula 1 is not only a new material for revitalizing functional cosmetics in the domestic cosmetics industry, but also a new compound derived from an amino acid structure, and may be a novel and multifunctional compound that exhibits an inhibitory efficacy on MMP-1 (Collagenase) and/or MMP-2 & MMP-9 (Gelatinases) at a low nanomolar (nM) concentration, for example at a concentration of 500 nM or lower, in a composition, for example a food composition or a cosmetic composition.In an embodiment, the compound represented by Formula 1 may be any one of the following compounds:

N-hydroxy-1-(phenylsulfonyl)piperidine-2-carboxamide,
1-([1,1'-biphenyl]-4-ylsulfonyl)-N-hydroxypiperidine-2-carboxamide,
1-((4-butoxyphenyl)sulfonyl)-N-hydroxypiperidine-2-carboxamide,
N-hydroxy-1-(mesitylsulfonyl)piperidine-2-carboxamide,
2-((4-(tert-butyl)phenyl)sulfonamido)-N-hydroxy-3-phenylpropanamide,
N-hydroxy-3-phenyl-2-((2,4,6-trimethylphenyl)sulfonamido)propanamide,

N-hydroxy-2-(naphthalene-2-sulfonamido)-3-phenylpropanamide,
2-([1,1'-biphenyl]-4-sulfonamido)-N-hydroxy-4-methylpentanamide,
2-((4-(tert-butyl)phenyl)sulfonamido)-N-hydroxy-4-methylpentanamide,
2-((4-butoxyphenyl)sulfonamido)-N-hydroxy-4-methylpentanamide,
N-hydroxy-4-methyl-2-((2,4,6-trimethylphenyl)sulfonamido)pentanamide,
2-((4-(tert-butyl)phenyl)sulfonamido)-N-hydroxy-3-methylbutanamide,
N-hydroxy-3-methyl-2-((2,4,6-trimethylphenyl)sulfonamido)butanamide,
1-((4-fluorophenyl)sulfonyl)-N-hydroxypiperidine-2-carboxamide,
1-((4-((3r,5r,7r)-adamantan-1-yl)phenyl)sulfonyl)-N-hydroxypiperidine-2-carboxamide,
N-hydroxy-1-((4-propylphenyl)sulfonyl)piperidine-2-carboxamide,
1-((4-acetamidophenyl)sulfonyl)-N-hydroxypiperidine-2-carboxamide,
1-((4-(tert-butyl)phenyl)sulfonyl)-N-hydroxypyrrolidine-2-carboxamide,
1-((4-butoxyphenyl)sulfonyl)-N-hydroxypyrrolidine-2-carboxamide,
1-((4-((3r,5r,7r)-adamantan-1-yl)phenyl)sulfonyl)-N-hydroxypyrrolidine-2-carboxamide,
N-hydroxy-1-((4-propylphenyl)sulfonyl)pyrrolidine-2-carboxamide,
1-((4-acetylphenyl)sulfonyl)-N-hydroxypyrrolidine-2-carboxamide,
N-hydroxy-1-((4-(1-(hydroxyimino)ethyl)phenyl)sulfonyl)piperidine-2-carboxamide,
N-hydroxy-4-methyl-2-(phenylsulfonamido)pentanamide, and
1-((4-acetamidophenyl)sulfonyl)-N-hydroxypyrrolidine-2-carboxamide.

In an embodiment, the compound represented by Formula 1 may be represented by any of the following formulas:

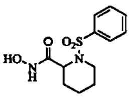 T-01 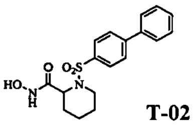 T-02 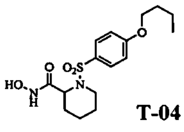 T-04 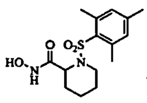 T-05 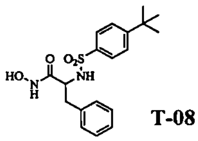 T-08

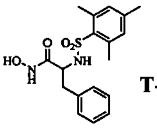 T-10 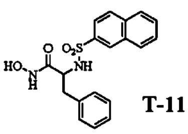 T-11 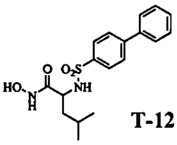 T-12 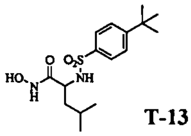 T-13 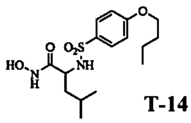 T-14

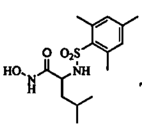 T-15 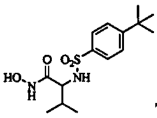 T-18 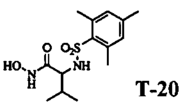 T-20 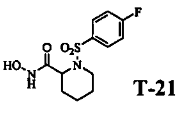 T-21 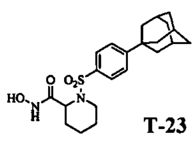 T-23

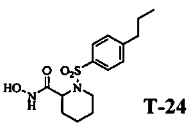 T-24 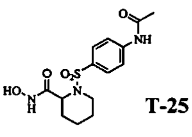 T-25 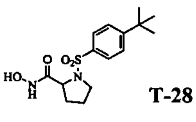 T-28 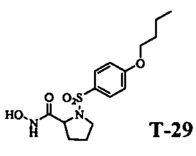 T-29 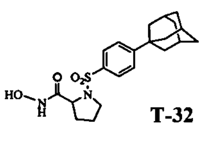 T-32

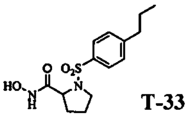 T-33 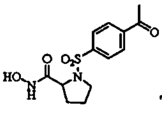 T-34 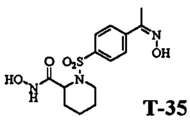 T-35 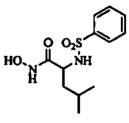 T-36 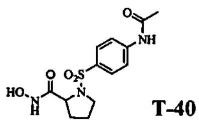 T-40

In an embodiment, the compound represented by Formula 1 may be derived from an amino acid.

For example, the compound represented by Formula 1 may be derived from any one of the following amino acids.

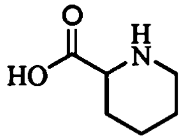

Pipecolinic acid

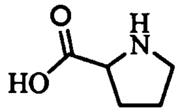

Proline

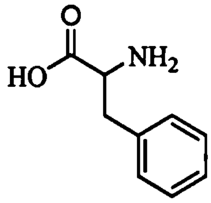

Phenylalanine

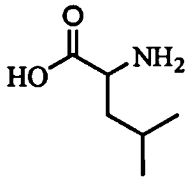

Leucine

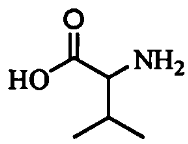

Valine

In still other exemplary embodiments of the present disclosure, there is provided a composition comprising at least one compound represented by formula 1, an isomer thereof, a pharmaceutically acceptable salt thereof, a hydrate thereof, or a solvate thereof, as an active ingredient.

In an embodiment, a concentration of the compound represented by Formula 1 in a composition, may be 0.01 μM to 100 mM based on the total volume of the composition.

For example, the concentration may be, based on the total volume of the composition, 0.01 μM or more, 0.05 μM or more, 0.1 μM or more, 1 μM or more, 10 μM or more, 100 μM or more, 0.5 mM or more, 1 mM or more, 1.5 mM or more, 2 mM or more, 2.5 mM or more, 10 mM or more, or 50 mM or more, and may be 100 mM or less, 50 mM or less, 10 mM or less, 8 mM or less, 6 mM or less, 4 mM or less, 1 mM or less, 100 μM or less, 10 μM or less, 1 μM or less, 0.1 μM or less, or 0.05 μM or less.

For example, the concentration may be, based on the total volume of the composition, 0.01 μM to 100 mM, preferably 0.05 μM to 10 mM, more preferably 0.1 μM to 4 mM.

If the above concentration is less than 0.01 μM, the efficacy may be minimal, and if it is more than 100 mM, there may be a cytotoxicity problem due to the high concentration.

In an embodiment, the composition may be to inhibit MMP-1 activity.

In an embodiment, the composition may be to inhibit MMP-2 activity.

The composition comprising the novel compound according to the present disclosure may be specifically intended to inhibit the MMP-2 activity. The MMP-2 is an enzyme (Gelatinase) that decomposes basement membrane

Collagen IV, so it is effective on the skin. An expression of the MMP-2 increases in proportion to an age and a photodamage, and plays an especially important role in the dermis, and is important to maintain and protect the Collage IV at a healthy level.

In an embodiment, the composition may be to inhibit MMP-9 activity.

In an embodiment, the composition may be a cosmetic composition comprising at least one compound represented by formula 1, an isomer thereof, a pharmaceutically acceptable salt thereof, a hydrate thereof, or a solvate thereof, as an active ingredient.

In an embodiment, an external form of the cosmetic composition may contain a cosmetically or dermatologically acceptable medium or substrate. The cosmetic composition may be provided in all formulations suitable for topical application, for example, in the form of an emulsion, a suspension, a microemulsion, a microcapsule, a microgranule, or an ionic (liposome) and non-ionic vesicular dispersion, which are obtained by dispersing an oil phase in a solution, a gel, a solid, a pasty anhydrous product, or a water phase, or in the form of a cream, a skin, a lotion, a powder, an ointment, a spray or a concealer stick. The composition can be prepared according to the conventional methods well known in the art.

The cosmetic composition according to the present disclosure can also be used in the form of a foam or in the form of an aerosol composition further comprising a compressed propellant.

The cosmetic composition according to the present disclosure is not particularly limited in formulation thereof, and can be, for example, formulated into cosmetics such as a softening lotion, an astringent lotion, a nourishing lotion, a nourishing cream, a massage cream, an essence, an eye cream, an eye essence, a cleansing cream, a cleansing foam, a cleansing water, a cleansing tissue containing the cosmetic composition, a pack, a powder, a body lotion, a body cream, a body oil, and a body essence.

In case the formulation of the cosmetic composition according to the present disclosure is a paste, a cream, or a gel, an animal fiber, a plant fiber, a wax, a paraffin, a starch, a tracant, a cellulose derivative, polyethylene glycol, a silicone, a bentonite, a silica, a talc, or zinc oxide may be used as a carrier ingredient.

In case the formulation of the cosmetic composition according to the present disclosure is a powder or a spray, a lactose, a talc, a silica, aluminum hydroxide, calcium silicate, or a polyamide powder may be used as the carrier ingredient, and especially in the case of the spray, the formulation may further contain the propellant such as chlorofluorohydrocarbon, propane/butane, or dimethyl ether.

In case the formulation of the cosmetic composition according to the present disclosure is a solution or an emulsion, a solvent, a solvating agent, or an emulsifying agent is used as the carrier ingredient, which include, for example, water, ethanol, isopropanol, ethyl carbonate, ethyl acetate, benzyl alcohol, benzyl benzoate, propylene glycol, 1,3-butylglycol oil, glycerol fatty ester, polyethylene glycol, or fatty acid ester of sorbitan.

In case the formulation of the cosmetic composition according to the present disclosure is a suspension, the carrier ingredient may include water, a liquid diluent such as ethanol or propylene glycol, a suspending agent such as ethoxylated isostearyl alcohol, polyoxyethylene sorbitol ester, and polyoxyethylene sorbitan ester, microcrystalline cellulose, aluminum metahydroxide, a bentonite, an agar, or a tracant.

The cosmetic composition according to the present disclosure may additionally comprise a functional additive and the components contained in general cosmetic compositions. The functional additive may include ingredients selected from the group consisting of a watersoluble vitamin, an oil-soluble vitamin, a high molecular weight peptide, a high molecular weight polysaccharide, a squalane, a sphingo lipid, and a seaweed extract.

In addition to the above-mentioned functional additive, the cosmetic composition according to the present disclosure may also comprise the components added to the general cosmetic composition, if necessary. Those components include an oil and fat ingredient, a moisturizer, an emollient, an emulsifier, organic and inorganic pigments, organic powders, an ultraviolet absorber, a preservative, a disinfectant, an antioxidant, a plant extract, a pH adjuster, an alcohol, a pigment, a fragrance, a blood circulation promoter, a coolant, a restrictor, a purified water, etc.

In an embodiment, the composition may be a food composition comprising at least one compound represented by formula 1, an isomer thereof, a pharmaceutically acceptable salt thereof, a hydrate thereof, or a solvate thereof, as an active ingredient.

The food composition according to the present disclosure may be formulated into a liquid or solid form, and may be in a formulation having the form of a tablet, a capsule, a soft capsule, a pill, a granule, a beverage (drink), a diet bar, a chocolate, a caramel, or confectionery, but are not particularly limited thereto. In addition to the above active ingredients, the food composition of the present disclosure may comprise an excipient, a saccharide, a flavoring, a pigment, a fat and oil, a protein, etc., if necessary.

In still other exemplary embodiments of the present disclosure, there is provided a method for preparing the compound represented by formula 1, comprising reacting an amino acid selected from a pipecolinic acid, a phenylalanine, leucine, a valine and a proline, and any one selected from a sulfonic acid, a sulfonyl halide, a sulfanilic acid, and a sulfanilyl halide.

In an embodiment, the sulfonyl halide may be a sulfonyl fluoride, a sulfonyl chloride, a sulfonyl bromide, or a sulfonyl iodide.

In an embodiment, the sulfanilyl halide may be a a sulfanylyl fluoride, a sulfanylyl chloride, a sulfanylyl bromide, or a sulfanylyl iodide.

In an embodiment, the method may further comprise obtaining the compound represented by formula 1 by reacting the intermediate obtained through the above reaction with a hydroxylamine(free form) or a hydroxylamine salt.

In an embodiment, the hydroxylamine salt may be a hydroxylaminechloride.

In an embodiment, the sulfonyl chloride may be any one selected from a group of a benzenesulfonyl chloride, 4-biphenylsulfonyl chloride, 4-butoxybenzene-1-sulfonyl chloride, 2-mesitylenesulfonyl chloride, 4-tert-butylbenzenesulfonyl chloride, 2-naphthalenesulfonyl chloride, 4-fluorobenzenesulfonyl chloride, 4-(1-adamantyl)benzenesulfonyl chloride, 4-n-propylbenzenesulfonyl chloride, and 4-acetylbenzenesulfonyl chloride, and the sulfanylyl chloride may be N-acetylsulfanylyl chloride.

In still other exemplary embodiments, the present disclosure provides a method of inhibiting the activity of MMP-1 that comprises administering a composition comprising, as an active ingredient, the compound represented by formula 1, an isomer thereof, a pharmaceutically acceptable salt thereof, a hydrate thereof, or a solvate thereof, in an effective amount to a subject in need thereof.

In still other exemplary embodiments, the present disclosure provides a method of inhibiting the activity of MMP-2 that comprises administering a composition comprising, as an active ingredient, the compound represented by formula 1, an isomer thereof, a pharmaceutically acceptable salt thereof, a hydrate thereof, or a solvate thereof, in an effective amount to a subject in need thereof.

In still other exemplary embodiments, the present disclosure provides a method of inhibiting the activity of MMP-9 that comprises administering a composition comprising, as an active ingredient, the compound represented by formula 1, an isomer thereof, a pharmaceutically acceptable salt thereof, a hydrate thereof, or a solvate thereof, in an effective amount to a subject in need thereof.

In still other exemplary embodiments, the present disclosure provides a use of the compound represented by formula 1, an isomer thereof, a pharmaceutically acceptable salt thereof, a hydrate thereof, or a solvate thereof, so as to prepare a composition for inhibiting the activity of MMP-1.

In still other exemplary embodiments, the present disclosure provides a use of the compound represented by formula 1, an isomer thereof, a pharmaceutically acceptable salt thereof, a hydrate thereof, or a solvate thereof, so as to prepare a composition for inhibiting the activity of MMP-2.

In still other exemplary embodiments, the present disclosure provides a use of the compound represented by formula 1, an isomer thereof, a pharmaceutically acceptable salt thereof, a hydrate thereof, or a solvate thereof, so as to prepare a composition for inhibiting the activity of MMP-9.

In still other exemplary embodiments, the present disclosure provides a non-therapeutic use of the compound represented by formula 1, an isomer thereof, a pharmaceutically acceptable salt thereof, a hydrate thereof, or a solvate thereof, to inhibit the activity of MMP-1.

In still other exemplary embodiments, the present disclosure provides a non-therapeutic use of the compound represented by formula 1, an isomer thereof, a pharmaceutically acceptable salt thereof, a hydrate thereof, or a solvate thereof, to inhibit the activity of MMP-2.

In still other exemplary embodiments, the present disclosure provides a non-therapeutic use of the compound represented by formula 1, an isomer thereof, a pharmaceutically acceptable salt thereof, a hydrate thereof, or a solvate thereof, to inhibit the activity of MMP-9.

Hereinafter, the present disclosure will be described in more detail through the following Examples. However, the following Examples are intended to provide only for illustrative purposes to aid understanding of the present disclosure, without limiting the scope of the present disclosure thereto.

## Example

### Preparation Example

New compounds were prepared as follows, and the IUPAC nomenclatures of the 25 new compounds are shown in Table 1 below.

[Table 1]

| TM No. | Compound Name |
|---|---|
| T-01 | N-hydroxy-1-(phenylsulfonyl)piperidine-2-carboxamide |
| T-02 | 1-([1,1'-biphenyl]-4-ylsulfonyl)-N-hydroxypiperidine-2-carboxamide |
| T-04 | 1-((4-butoxyphenyl)sulfonyl)-N-hydroxypiperidine-2-carboxamide |

(continued)

| TM No. | Compound Name |
|---|---|
| T-05 | N-hydroxy-1-(mesitylsulfonyl)piperidine-2-carboxamide |
| T-08 | 2-((4-(tert-butyl)phenyl)sulfonamido)-N-hydroxy-3-phenylpropanamide |
| T-10 | N-hydroxy-3-phenyl-2-((2,4,6-trimethylphenyl)sulfonamido)propanamide |
| T-11 | N-hydroxy-2-(naphthalene-2-sulfonamido)-3-phenylpropanamide |
| T-12 | 2-([1,1'-biphenyl]-4-sulfonamido)-N-hydroxy-4-methylpentanamide |
| T-13 | 2-((4-(tert-butyl)phenyl)sulfonamido)-N-hydroxy-4-methylpentanamide |
| T-14 | 2-((4-butoxyphenyl)sulfonamido)-N-hydroxy-4-methylpentanamide |
| T-15 | N-hydroxy-4-methyl-2-((2,4,6-trimethylphenyl)sulfonamido)pentanamide |
| T-18 | 2-((4-(tert-butyl)phenyl)sulfonamido)-N-hydroxy-3-methylbutanamide |
| T-20 | N-hydroxy-3-methyl-2-((2,4,6-trimethylphenyl)sulfonamido)butanamide |
| T-21 | 1-((4-fluorophenyl)sulfonyl)-N-hydroxypiperidine-2-carboxamide |
| T-23 | 1-((4-((3r,5r,7r)-adamantan-1-yl)phenyl)sulfonyl)-N-hydroxypiperidine-2-carboxamide |
| T-24 | N-hydroxy-1-((4-propylphenyl)sulfonyl)piperidine-2-carboxamide |
| T-25 | 1-((4-acetamidophenyl)sulfonyl)-N-hydroxypiperidine-2-carboxamide |
| T-28 | 1-((4-(tert-butyl)phenyl)sulfonyl)-N-hydroxypyrrolidine-2-carboxamide |
| T-29 | 1-((4-butoxyphenyl)sulfonyl)-N-hydroxypyrrolidine-2-carboxamide |
| T-32 | 1-((4-((3r,5r,7r)-adamantan-1-yl)phenyl)sulfonyl)-N-hydroxypyrrolidine-2-carboxamide |
| T-33 | N-hydroxy-1-((4-propylphenyl)sulfonyl)pyrrolidine-2-carboxamide |
| T-34 | 1-((4-acetylphenyl)sulfonyl)-N-hydroxypyrrolidine-2-carboxamide |
| T-35 | N-hydroxy-1-((4-(1-(hydroxyimino)ethyl)phenyl)sulfonyl)piperidine-2-carboxamide |
| T-36 | N-hydroxy-4-methyl-2-(phenylsulfonamido)pentanamide |
| T-40 | 1-((4-acetamidophenyl)sulfonyl)-N-hydroxypyrrolidine-2-carboxamide |

**(1) T-01** Synthesis of N-hydroxy-1-(phenylsulfonyl)piperidine-2-carboxamide

1.69 g of pipecolinic acid was well stirred with 30 mL of THF, and then 30 mL of 1M $Na_2CO_3$ was added thereto. 2 mL of benzenesulfonyl chloride was slowly added dropwise at 0 °C and stirred at the same temperature for 1 hour. After the mixture was washed with 30 mL of diethyl ether, an aqueous layer was adjusted to pH 3 with 3M HCl. After the aqueous layer was extracted with ethyl acetate, it was dried using $MgSO_4$ and concentrated under reduced pressure to obtain 1.89 g of 1-(phenylsulfonyl)piperidine-2-carboxylic acid as an intermediate. The intermediate was used in the next reaction without any additional purification process.

1.89 g of 1-(phenylsulfonyl)piperidine-2-carboxylic acid was dissolved in 15 mL of THF along with 0.85 mL of NMO (N-methylmorpholine-N-oxide) and stirred at 0 °C. 0.73 mL of ethyl chloroformate was slowly added dropwise and stirred at the same temperature for 30 minutes. After solids were filtered from the reaction solution and the obtained filtrate was stored separately, 0.73 g of hydroxylamine hydrochloride and 1.46 mL of triethylamine were added dropwise to a solution dissolved in 20 mL of DMF. After stirring at a room temperature for 30 minutes, the DMF was removed by concentration under reduced pressure. After the layers were separated by adding ethyl acetate and purified water, the aqueous layer was extracted twice more using ethyl acetate. After the organic layer was collected and concentrated under reduced pressure, the obtained solids were purified by a column chromatography (hexane: ethyl acetate = 1:2) to produce 0.72 g of N-hydroxy-1-(phenylsulfonyl)piperidine-2-carboxamide.
$^1$H-NMR (500 MHz, DMSO-$d_6$): δ 10.62 (s, 1H), 8.79 (s, 1H), 7.78 (m, 2H), 7.66 (m, 1H), 7.57 (m, 2H), 4.34 (m, 1H), 3.63 (m, 1H), 3.47 (m, 1H), 1.78 (m, 1H), 1.55 (m, 1H) 1.45 - 1.40 (m, 3H), 1.17 (m, 1H)

**(2) T-02** Synthesis of 1-([1,1'-biphenyl]-4-ylsulfonyl)-N-hydroxypiperidine-2-carboxamide

0.65 g of pipecolinic acid was well stirred with 20 mL of THF, and then 11.5 mL of 1M $Na_2CO_3$ was added thereto.

1.52 g of 4-biphenylsulfonyl chloride was slowly added dropwise at 0 °C and stirred at a room temperature for 1 hour. After the mixture was washed twice with 50 mL of diethyl ether, an aqueous layer was adjusted to pH 3 with 3M HCl. After the aqueous layer was extracted with ethyl acetate, it was dried using $MgSO_4$ and concentrated under reduced pressure to obtain 0.78 g of 1-([1,1'-biphenyl]-4-ylsulfonyl)piperidine-2-carboxylic acid as an intermediate. The intermediate was used in the next reaction without any additional purification process.

0.7 g of 1-([1,1'-biphenyl]-4-ylsulfonyl)piperidine-2-carboxylic acid was dissolved in 20 mL of THF along with 0.25 mL of NMO (N-methylmorpholine-N-oxide) and stirred at 0 °C. 0.21 mL of ethyl chloroformate was slowly added dropwise and stirred at the same temperature for 30 minutes. After solids were filtered from the reaction solution and the obtained filtrate was stored separately, 0.21 g of hydroxylamine hydrochloride and 0.42 mL of triethylamine were added dropwise to a solution dissolved in 10 mL of DMF. After stirring at a room temperature for 30 minutes, the DMF was removed by concentration under reduced pressure. After the layers were separated by adding ethyl acetate and purified water, the aqueous layer was extracted twice more using ethyl acetate. After the organic layer was collected and concentrated under reduced pressure, the obtained solids were purified by a column chromatography (hexane: ethyl acetate = 1:2) to produce 0.26 g of 1-([1,1'-biphenyl]-4-ylsulfonyl)-N-hydroxypiperidine-2-carboxamide.

$^1$H-NMR (500 MHz, DMSO-$d_6$): δ 10.67 (s, 1H), 8.80 (s, 1H), 7.87 (m, 4H), 7.77 (m, 2H), 7.52 (m, 2H), 7.45 (m, 1H), 4.38 (m, 1H), 3.66 (m, 1H), 3.50 (m, 1H), 1.81 (m, 1H), 1.59 (m, 1H), 1.47-1.43 (m, 3H), 1.26-1.15 (m, 1H)

**(3) T-04** Synthesis of 1-((4-butoxyphenyl)sulfonyl)-N-hydroxypiperidine-2-carboxamide

0.65 g of pipecolinic acid was well stirred with 20 mL of THF, and then 11.5 mL of 1M $Na_2CO_3$ was added thereto. 1.2 mL of 4-butoxybenzene-1-sulfonylchloride was slowly added dropwise at 0 °C and stirred at a room temperature for 1 hour. After the mixture was washed twice with 50 mL of diethyl ether, an aqueous layer was adjusted to pH 3 with 3M HCl. After the aqueous layer was extracted with 100 mL of ethyl acetate, it was dried using $MgSO_4$ and concentrated under reduced pressure to obtain 1.17 g of 1-((4-butoxyphenyl)sulfonyl)piperidine-2-carboxylic acid as an intermediate. The intermediate was used in the next reaction without any additional purification process.

1.17 g of 1-((4-butoxyphenyl)sulfonyl)piperidine-2-carboxylic acid was dissolved in 30 mL of THF along with 0.42 mL of NMO (N-methylmorpholine-N-oxide) and stirred at 0 °C. 0.36 mL of ethyl chloroformate was slowly added dropwise and stirred at the same temperature for 30 minutes. After solids were filtered from the reaction solution and the obtained filtrate was stored separately, 0.36 g of hydroxylamine hydrochloride and 0.72 mL of triethylamine were added dropwise to a solution dissolved in 20 mL of DMF. After stirring at a room temperature for 30 minutes, the DMF was removed by concentration under reduced pressure. After the layers were separated by adding ethyl acetate and purified water, the aqueous layer was extracted twice more using ethyl acetate. After the organic layer was collected and concentrated under reduced pressure, the obtained solids were purified by a column chromatography (hexane: ethyl acetate = 1:1) to produce 0.37 g of 1-((4-butoxyphenyl)sulfonyl)-N- hydroxypiperidine-2-carboxamide

$^1$H-NMR (500 MHz, DMSO-$d_6$): δ 10.61 (s, 1H), 8.77 (s, 1H), 7.67 (d, 2H, *J*=8.5Hz), 7.06 (d, 2H, *J*=9Hz), 4.30 (m, 1H), 4.05 (t, 2H, *J*=7Hz), 3.59 (m, 1H), 3.44 (m, 1H) 1.77-1.69 (m, 3H), 1.54-1.42 (m, 6H), 1.18 (m, 1H), 0.94 (t, 3H, *J*=7.5Hz)

**(4) T-05** Synthesis of N-hydroxy-1-(mesitylsulfonyl)piperidine-2-carboxamide

0.65 g of pipecolinic acid was well stirred with 20 mL of THF, and then 11.5 mL of 1M $Na_2CO_3$ was added thereto. 1.31 g of 2-mesitylenesulfonyl chloride was slowly added dropwise at 0 °C and stirred at a room temperature for 1 hour. After the mixture was washed twice with 50 mL of diethyl ether, an aqueous layer was adjusted to pH 3 with 3M HCl. After the aqueous layer was extracted with 100 mL of ethyl acetate, it was dried using $MgSO_4$ and concentrated under reduced pressure to obtain 0.71 g of 1-(mesitylsulfonyl)piperidine-2-carboxylic acid as an intermediate. The intermediate was used in the next reaction without any additional purification process.

0.69 g of 1-(mesitylsulfonyl)piperidine-2-carboxylic acid was dissolved in 20 mL of THF along with 0.27 mL of NMO (N-methylmorpholine-N-oxide) and stirred at 0 °C. 0.23 mL of ethyl chloroformate was slowly added dropwise and stirred at the same temperature for 30 minutes. After solids were filtered from the reaction solution and the obtained filtrate was stored separately, 0.23 g of hydroxylamine hydrochloride and 0.46 mL of triethylamine were added dropwise to a solution dissolved in 20 mL of DMF. After stirring at a room temperature for 30 minutes, the DMF was removed by concentration under reduced pressure. After the layers were separated by adding ethyl acetate and purified water, the aqueous layer was extracted twice more using ethyl acetate. After the organic layer was collected and concentrated under reduced pressure, the obtained solids were purified by a column chromatography (hexane: ethyl acetate = 1:1) to produce 0.14 g of N-hydroxy-1-(mesitylsulfonyl)piperidine-2-carboxamide.

$^1$H-NMR (500 MHz, DMSO-$d_6$): δ 10.48 (s, 1H), 8.86 (s, 1H), 7.05 (s, 2H), 3.69 (t, 1H, *J*=10.5Hz), 3.32 (s, 3H), 3.43 (s, 3H) 1.79 (m, 2H), 1.65-1.50 (m, 6H), 1.30-1.23 (m, 3H)

**(5) T-08** Synthesis of 2-((4-(tert-butyl)phenyl)sulfonamido)-N-hydroxy-3-phenylpropanamide

0.83 g of phenylalanine was well stirred with 20 mL of THF, and then 11.5 mL of 1M $Na_2CO_3$ was added thereto. 1.4 g of 4-tert-butylbenzenesulfonyl chloride was slowly added dropwise at 0 °C and stirred at a room temperature for 1 hour. After the mixture was washed twice with 50 mL of diethyl ether, an aqueous layer was adjusted to pH 3 with 3M HCl. After the aqueous layer was extracted with 100 mL of ethyl acetate, it was dried using $MgSO_4$ and concentrated under reduced pressure to obtain 1.31 g of ((4-(tert-butyl)phenyl)sulfonyl)phenylalanine as an intermediate. The intermediate was used in the next reaction without any additional purification process.

1.2 g of ((4-(tert-butyl)phenyl)sulfonyl)phenylalanine was dissolved in 20 mL of THF along with 0.4 mL of NMO (N-methylmorpholine-N-oxide) and stirred at 0 °C. 0.35 mL of ethyl chloroformate was slowly added dropwise and stirred at the same temperature for 30 minutes. After solids were filtered from the reaction solution and the obtained filtrate was stored separately, 0.35 g of hydroxylamine hydrochloride and 0.69 mL of triethylamine were added dropwise to a solution dissolved in 20 mL of DMF. After stirring at a room temperature for 30 minutes, the DMF was removed by concentration under reduced pressure. After the layers were separated by adding ethyl acetate and purified water, the aqueous layer was extracted twice more using ethyl acetate. After the organic layer was collected and concentrated under reduced pressure, the obtained solids were purified by a column chromatography (hexane: ethyl acetate = 1:1) to produce 0.38 g of 2-((4-(tert-butyl)phenyl)sulfonamido)-N-hydroxy-3-phenylpropanamide.

$^1$H-NMR (500 MHz, DMSO-$d_6$): δ 10.61 (s, 1H), 8.88 (s, 1H), 8.13 (d, 1H, $J$=8.5Hz), 7.40 (m, 4H), 7.12 (m, 3H), 7.00 (m, 2H), 3.71 (m, 1H), 2.76 (m, 1H), 2.55 (m, 1H), 1.26 (s, 9H)

**(6) T-10** Synthesis of N-hydroxy-3-phenyl-2-((2,4,6-trimethylphenyl)sulfonamido)propanamide

0.83 g of phenylalanine was well stirred with 20 mL of THF, and then 11.5 mL of 1M $Na_2CO_3$ was added thereto. 1.31 g of 2-mesitylenesulfonyl chloride was slowly added dropwise at 0 °C and stirred at a room temperature for 1 hour. After the mixture was washed twice with 50 mL of diethyl ether, an aqueous layer was adjusted to pH 3 with 3M HCl. After the aqueous layer was extracted with 100 mL of ethyl acetate, it was dried using $MgSO_4$ and concentrated under reduced pressure to obtain 1 g of (mesitylsulfonyl)phenylalanine as an intermediate. The intermediate was used in the next reaction without any additional purification process.

1 g of (mesitylsulfonyl)phenylalanine was dissolved in 20 mL of THF along with 0.35 mL of NMO (N-methylmorpholine-N-oxide) and stirred at 0 °C. 0.3 mL of ethyl chloroformate was slowly added dropwise and stirred at the same temperature for 30 minutes. After solids were filtered from the reaction solution and the obtained filtrate was stored separately, 0.3 g of hydroxylamine hydrochloride and 0.6 mL of triethylamine were added dropwise to a solution dissolved in 20 mL of DMF. After stirring at a room temperature for 30 minutes, the DMF was removed by concentration under reduced pressure. After the layers were separated by adding ethyl acetate and purified water, the aqueous layer was extracted twice more using ethyl acetate. After the organic layer was collected and concentrated under reduced pressure, the obtained solids were purified by a column chromatography (hexane: ethyl acetate = 1:1) to produce 0.25 g of N-hydroxy-3-phenyl-2-((2,4,6-trimethylphenyl)sulfonamido)propenamide.

$^1$H-NMR (500 MHz, DMSO-$d_6$): δ 10.59 (s, 1H), 8.87 (s, 1H), 7.89 (d, 1H, $J$=9.5Hz), 7.07 (m, 3H), 6.95 (m, 2H), 6.82 (m, 2H), 3.67 (m, 1H), 2.76 (m, 1H), 2.60 (m, 1H), 2.49 (s, 6H), 2.26 (s, 3H)

**(7) T-11** Synthesis of N-hydroxy-2-(naphthalene-2-sulfonamido)-3-phenylpropanamide

0.83 g of phenylalanine was well stirred with 20 mL of THF, and then 11.5 mL of 1M $Na_2CO_3$ was added thereto. 1.36 g of 2-naphthalenesulfonyl chloride was slowly added dropwise at 0 °C and stirred at a room temperature for 1 hour. After the mixture was washed twice with 50 mL of diethyl ether, an aqueous layer was adjusted to pH 3 with 3M HCl. After the aqueous layer was extracted with 100 mL of ethyl acetate, it was dried using $MgSO_4$ and concentrated under reduced pressure to obtain 1.2 g of (naphthalen-2-ylsulfonyl)phenylalanine as an intermediate. The intermediate was used in the next reaction without any additional purification process.

1.1 g of (naphthalen-2-ylsulfonyl)phenylalanine was dissolved in 20 mL of THF along with 0.37 mL of NMO (N-methylmorpholine-N-oxide) and stirred at 0 °C. 0.32 mL of ethyl chloroformate was slowly added dropwise and stirred at the same temperature for 30 minutes. After solids were filtered from the reaction solution and the obtained filtrate was stored separately, 0.32 g of hydroxylamine hydrochloride and 0.65 mL of triethylamine were added dropwise to a solution dissolved in 20 mL of DMF. After stirring at a room temperature for 30 minutes, the DMF was removed by concentration under reduced pressure. After the layers were separated by adding ethyl acetate and purified water, the aqueous layer was extracted twice more using ethyl acetate. After the organic layer was collected and concentrated under reduced pressure, the obtained solids were purified by a column chromatography (hexane: ethyl acetate = 1:2) to produce 0.21 g of N-hydroxy-2-(naphthalene-2-sulfonamido)-3-phenylpropanamide.

$^1$H-NMR (500 MHz, DMSO-$d_6$): δ 10.63 (s, 1H), 8.84 (s, 1H), 8.32 (d, 1H, $J$=9.5Hz), 8.19 (s, 1H), 8.19 (m, 2H), 7.92 (d, 1H,

*J*=9.5Hz), 7.65 (m, 2H), 7.56 (m, 1H), 7.28-7.00 (m, 5H), 3.84 (q, 1H), 2.78 (m, 1H), 2.59 (m, 1H)

**(8) T-12** Synthesis of 2-([1,1'-biphenyl]-4-sulfonamido)-N-hydroxy-4-methylpentanamide

0.66 g of leucine was well stirred with 20 mL of THF, and then 11.5 mL of 1M $Na_2CO_3$ was added thereto. 1.52 g of 4-biphenylsulfonyl chloride was slowly added dropwise at 0 °C and stirred at a room temperature for 1 hour. After the mixture was washed twice with 50 mL of diethyl ether, an aqueous layer was adjusted to pH 3 with 3M HCl. After the aqueous layer was extracted with 100 mL of ethyl acetate, it was dried using $MgSO_4$ and concentrated under reduced pressure to obtain 0.64 g of ([1,1'-biphenyl]-4-ylsulfonyl)leucine as an intermediate. The intermediate was used in the next reaction without any additional purification process.

0.6 g of ([1,1'-biphenyl]-4-ylsulfonyl)leucine was dissolved in 20 mL of THF along with 0.21 mL of NMO (N-methylmorpholine-N-oxide) and stirred at 0 °C. 0.18 mL of ethyl chloroformate was slowly added dropwise and stirred at the same temperature for 30 minutes. After solids were filtered from the reaction solution and the obtained filtrate was stored separately, 0.18 g of hydroxylamine hydrochloride and 0.36 mL of triethylamine were added dropwise to a solution dissolved in 10 mL of DMF. After stirring at a room temperature for 30 minutes, the DMF was removed by concentration under reduced pressure. After the layers were separated by adding ethyl acetate and purified water, the aqueous layer was extracted twice more using ethyl acetate. After the organic layer was collected and concentrated under reduced pressure, the obtained solids were purified by a column chromatography (hexane: ethyl acetate = 1:1) to produce 0.34 g of 2-([1,1'-biphenyl]-4-sulfonamido)-N-hydroxy-4-methylpentanamide.

$^1$H-NMR (500 MHz, DMSO-$d_6$): δ 10.66 (s, 1H), 8.81 (s, 1H), 8.03 (d, 1H, *J*=6.0Hz), 7.83 (m, 4H), 7.73 (d, 2H, *J*=7.0Hz), 7.51 (m, 2H), 7.43 (m, 1H), 3.59 (q, 1H), 1.41 (m, 1H), 1.33-1.21 (m, 2H), 0.75 (d, 3H, *J*=6.5Hz), 0.64 (d, 3H, *J*=7.0Hz)

**(9) T-13** Synthesis of 2-((4-(tert-butyl)phenyl)sulfonamido)-N-hydroxy-4-methylpentanamide

0.66 g of leucine was well stirred with 20 mL of THF, and then 11.5 mL of 1M $Na_2CO_3$ was added thereto. 1.4 g of 4-tert-butylbenzenesulfonyl chloride was slowly added dropwise at 0 °C and stirred at a room temperature for 1 hour. After the mixture was washed twice with 50 mL of diethyl ether, an aqueous layer was adjusted to pH 3 with 3M HCl. After the aqueous layer was extracted with 100 mL of ethyl acetate, it was dried using $MgSO_4$ and concentrated under reduced pressure to obtain 1.09 g of ((4-(tert-butyl)phenyl)sulfonyl)leucine as an intermediate. The intermediate was used in the next reaction without any additional purification process.

1.07 g of ((4-(tert-butyl)phenyl)sulfonyl)leucine was dissolved in 20 mL of THF along with 0.4 mL of NMO (N-methylmorpholine-N-oxide) and stirred at 0 °C. 0.35 mL of ethyl chloroformate was slowly added dropwise and stirred at the same temperature for 30 minutes. After solids were filtered from the reaction solution and the obtained filtrate was stored separately, 0.35 g of hydroxylamine hydrochloride and 0.7 mL of triethylamine were added dropwise to a solution dissolved in 20 mL of DMF. After stirring at a room temperature for 30 minutes, the DMF was removed by concentration under reduced pressure. After the layers were separated by adding ethyl acetate and purified water, the aqueous layer was extracted twice more using ethyl acetate. After the organic layer was collected and concentrated under reduced pressure, the obtained solids were purified by a column chromatography (hexane: ethyl acetate = 1:1) to produce 0.15 g of 2-((4-(tert-butyl)phenyl)sulfonamido)-N-hydroxy-4-methylpentanamide.

$^1$H-NMR (500 MHz, DMSO-$d_6$): δ 10.64 (s, 1H), 8.83 (s, 1H), 7.88 (m, 1H), 7.70 (d, 2H, *J*=9.0Hz), 7.57 (d, 2H, *J*=8.5Hz), 3.49 (m, 1H), 1.32-1.24 (m, 11H), 1.12 (m, 1H), 0.69 (d, 3H, *J*=6.5Hz), 0.55 (d, 3H, *J*=6.5Hz)

**(10) T-14** Synthesis of 2-((4-butoxyphenyl)sulfonamido)-N-hydroxy-4-methylpentanamide

0.66 g of leucine was well stirred with 20 mL of THF, and then 11.5 mL of 1M $Na_2CO_3$ was added thereto. 1.2 mL of 4-butoxybenzene-1-sulfonyl chloride was slowly added dropwise at 0 °C and stirred at a room temperature for 1 hour. After the mixture was washed twice with 50 mL of diethyl ether, an aqueous layer was adjusted to pH 3 with 3M HCl. After the aqueous layer was extracted with 100 mL of ethyl acetate, it was dried using $MgSO_4$ and concentrated under reduced pressure to obtain 1 g of ((4-butoxyphenyl)sulfonyl)leucine as an intermediate. The intermediate was used in the next reaction without any additional purification process.

1 g of ((4-butoxyphenyl)sulfonyl)leucine was dissolved in 20 mL of THF along with 0.35 mL of NMO (N-methylmorpholine-N-oxide) and stirred at 0 °C. 0.3 mL of ethyl chloroformate was slowly added dropwise and stirred at the same temperature for 30 minutes. After solids were filtered from the reaction solution and the obtained filtrate was stored separately, 0.3 g of hydroxylamine hydrochloride and 0.6 mL of triethylamine were added dropwise to a solution dissolved in 20 mL of DMF. After stirring at a room temperature for 30 minutes, the DMF was removed by concentration under reduced pressure. After the layers were separated by adding ethyl acetate and purified water, the aqueous layer was extracted twice more using ethyl acetate. After the organic layer was collected and concentrated under reduced pressure, the obtained solids were purified by a column chromatography (hexane: ethyl acetate = 1:1) to produce 40 mg of 2-((4-butoxyphenyl)sulfonamido)-N-hydroxy-4-methylpentanamide.
$^1$H-NMR (500 MHz, DMSO-*d$_6$*): δ 10.61 (s, 1H), 8.77 (s, 1H), 7.76 (m, 1H), 7.67 (d, 2H, *J*=9.0Hz), 7.04 (d, 2H, *J*=8.5Hz), 4.04 (t, 2H, *J*=6.5Hz), 3.50 (m, 1H), 1.70 (m, 2H), 1.45 (m, 3H), 1.27 (m, 1H), 1.19 (m, 1H), 0.93 (t, 3H, *J*=7.0Hz), 0.77-0.72 (d, 3H, *J*=6.5Hz), 0.63 (d, 3H, *J*=6.0Hz)

**(11) T-15** Synthesis of N-hydroxy-4-methyl-2-((2,4,6-trimethylphenyl)sulfonamido)pentanamide

0.66 g of leucine was well stirred with 20 mL of THF, and then 11.5 mL of 1M $Na_2CO_3$ was added thereto. 1.31 g of 2-mesitylenesulfonyl chloride was slowly added dropwise at 0 °C and stirred at a room temperature for 1 hour. After the mixture was washed twice with 50 mL of diethyl ether, an aqueous layer was adjusted to pH 3 with 3M HCl. After the aqueous layer was extracted with 100 mL of ethyl acetate, it was dried using $MgSO_4$ and concentrated under reduced pressure to obtain 0.76 g of (mesitylsulfonyl)leucine as an intermediate. The intermediate was used in the next reaction without any additional purification process.

0.75g of (mesitylsulfonyl)leucine was dissolved in 20 mL of THF along with 0.29 mL of NMO (N-methylmorpholine-N-oxide) and stirred at 0 °C. 0.25 mL of ethyl chloroformate was slowly added dropwise and stirred at the same temperature for 30 minutes. After solids were filtered from the reaction solution and the obtained filtrate was stored separately, 0.25 g of hydroxylamine hydrochloride and 0.5 mL of triethylamine were added dropwise to a solution dissolved in 20 mL of DMF. After stirring at a room temperature for 30 minutes, the DMF was removed by concentration under reduced pressure. After the layers were separated by adding ethyl acetate and purified water, the aqueous layer was extracted twice more using ethyl acetate. After the organic layer was collected and concentrated under reduced pressure, the obtained solids were purified by a column chromatography (hexane: ethyl acetate = 1:1) to produce 0.14 g of N-hydroxy-4-methyl-2-((2,4,6-trimethylphenyl)sulfonamido)pentanamide.
$^1$H-NMR (500 MHz, DMSO-*d$_6$*): δ 10.51 (s, 1H), 8.88 (s, 1H), 7.77 (m, 1H), 6.98 (s, 2H), 3.45 (m, 1H), 2.55 (s, 3H), 2.50 (s, 3H), 2.24 (s, 3H), 1.41 (m, 1H), 1.33-1.20 (m, 2H), 0.73 (d, 3H, *J*=6.5Hz), 0.55 (d, 3H, *J*=6.5Hz)

**(12) T-18** Synthesis of 2-((4-(tert-butyl)phenyl)sulfonamido)-N-hydroxy-3-methylbutanamide

0.59 g of valine was well stirred with 20 mL of THF, and then 11.5 mL of 1M $Na_2CO_3$ was added thereto. 1.4 g of 4-tert-butylbenzenesulfonyl chloride was slowly added dropwise at 0 °C and stirred at a room temperature for 1 hour. After the mixture was washed twice with 50 mL of diethyl ether, an aqueous layer was adjusted to pH 3 with 3M HCl. After the aqueous layer was extracted with 100 mL of ethyl acetate, it was dried using $MgSO_4$ and concentrated under reduced pressure to obtain 0.71 g of ((4-(tert-butyl)phenyl)sulfonyl)valine as an intermediate. The intermediate was used in the next reaction without any additional purification process.

0.7 g of ((4-(tert-butyl)phenyl)sulfonyl)valine was dissolved in 20 mL of THF along with 0.25 mL of NMO (N-methylmorpholine-N-oxide) and stirred at 0 °C. 0.21 mL of ethyl chloroformate was slowly added dropwise and stirred at the same temperature for 30 minutes. After solids were filtered from the reaction solution and the obtained filtrate was stored separately, 0.21 g of hydroxylamine hydrochloride and 0.42 mL of triethylamine were added dropwise to a solution dissolved in 20 mL of DMF. After stirring at a room temperature for 30 minutes, the DMF was removed by concentration under reduced pressure. After the layers were separated by adding ethyl acetate and purified water, the aqueous layer was extracted twice more using ethyl acetate. After the organic layer was collected and concentrated under reduced pressure, the obtained solids were purified by a column chromatography (hexane: ethyl acetate = 1:1) to produce 0.3 g of 2-((4-(tert-butyl)phenyl)sulfonamido)-N-hydroxy-3-methylbutanamide.
$^1$H-NMR (500 MHz, DMSO-*d$_6$*): δ 10.50 (s, 1H), 8.80 (s, 1H), 7.81 (m, 1H), 7.68 (d, 2H, *J*=8.0Hz), 7.54 (d, 2H, *J*=8.0Hz), 3.27 (m, 1H), 1.75 (m, 1H), 1.29 (s, 9H), 0.73 (d, 3H, *J*=6.5Hz), 0.70 (d, 3H, *J*=6.5Hz)

**(13) T-20** Synthesis of N-hydroxy-3-methyl-2-((2,4,6-trimethylphenyl)sulfonamido)butanamide

0.59 g of valine was well stirred with 20 mL of THF, and then 11.5 mL of 1M $Na_2CO_3$ was added thereto. 1.31 g of 2-mesitylenesulfonyl chloride was slowly added dropwise at 0 °C and stirred at a room temperature for 1 hour. After the mixture was washed twice with 50 mL of diethyl ether, an aqueous layer was adjusted to pH 3 with 3M HCl. After the aqueous layer was extracted with 100 mL of ethyl acetate, it was dried using $MgSO_4$ and concentrated under reduced pressure to obtain 0.53 g of (mesitylsulfonyl)valine as an intermediate. The intermediate was used in the next reaction without any additional purification process.

0.52 g of (mesitylsulfonyl)valine was dissolved in 20 mL of THF along with 0.25 mL of NMO (N-methylmorpholine-N-oxide) and stirred at 0 °C. 0.21 mL of ethyl chloroformate was slowly added dropwise and stirred at the same temperature for 30 minutes. After solids were filtered from the reaction solution and the obtained filtrate was stored separately, 0.21 g of hydroxylamine hydrochloride and 0.42 mL of triethylamine were added dropwise to a solution dissolved in 20 mL of DMF. After stirring at a room temperature for 30 minutes, the DMF was removed by concentration

under reduced pressure. After the layers were separated by adding ethyl acetate and purified water, the aqueous layer was extracted twice more using ethyl acetate. After the organic layer was collected and concentrated under reduced pressure, the obtained solids were purified by a column chromatography (hexane: ethyl acetate = 1:1) to produce 0.14 g of N-hydroxy-3-methyl-2-((2,4,6-trimethylphenyl)sulfonamido)butanamide.
$^1$H-NMR (500 MHz, DMSO-*d*$_6$): δ 10.43 (s, 1H), 8.80 (s, 1H), 7.59 (d, 1H, *J*=9.5Hz), 6.98 (s, 2H), 3.18 (t, 1H, *J*=8.0Hz), 2.59-2.50 (m, 6H), 2.24 (s, 3H), 1.76 (m, 1H), 0.70 (m, 6H)

**(14) T-21** Synthesis of 1-((4-fluorophenyl)sulfonyl)-N-hydroxypiperidine-2-carboxamide

500 mg of pipecolinic acid was well stirred with 8 mL of THF, and then 9.6 mL of 1M $Na_2CO_3$ was added thereto. 904 mg of 4-fluorobenzenesulfonyl chloride was slowly added dropwise at 0 °C and stirred at a room temperature for 2 hours. After the mixture was washed twice with 20 mL of diethyl ether, an aqueous layer was adjusted to pH 2 with 3M HCl. After the aqueous layer was extracted twice with 20 mL of ethyl acetate, it was dried using $MgSO_4$ and concentrated under reduced pressure to obtain 608 mg of 1-((4-fluorophenyl)sulfonyl)piperidine-2-carboxylic acid as an intermediate. The intermediate was used in the next reaction without any additional purification process.

550 mg of 1-((4-fluorophenyl)sulfonyl)piperidine-2-carboxylic acid was dissolved in 6 mL of THF along with 0.23 mL of NMO (N-methylmorpholine-N-oxide) and stirred at 0 °C. 0.2 mL of ethyl chloroformate was slowly added dropwise and stirred at the same temperature for 2 hours. After solids were filtered from the reaction solution and the obtained filtrate was stored separately, 0.2 g of hydroxylamine hydrochloride and 0.4 mL of triethylamine were added dropwise to a solution dissolved in 2 mL of DMF. After stirring at a room temperature for 2 hours, the DMF was removed by concentration under reduced pressure. After the layers were separated by adding ethyl acetate and purified water, the aqueous layer was extracted twice more using ethyl acetate. After the organic layer was collected and concentrated under reduced pressure, the obtained solids were purified by a column chromatography (5 % MeOH in DCM) to produce 0.14 g of 1-((4-fluorophenyl)sulfonyl)-N-hydroxypiperidine-2-carboxamide.
$^1$H-NMR (500 MHz, DMSO-*d*$_6$): δ 10.63 (s, 1H), 8.76 (s, 1H), 7.82 (m, 2H), 7.42 (m, 2H), 4.32 (m, 1H), 3.62 (m, 1H), 3.46 (m, 1H), 1.80 (m, 1H), 1.77 (m, 1H), 1.51-1.39 (m, 3H), 1.19 (m, 1H)

**(15) T-23** Synthesis of 1-((4-((3r,5r,7r)-adamantan-1-yl)phenyl)sulfonyl)-N-hydroxypiperidine-2-carboxamide

50 mg of pipecolinic acid was well stirred with 0.8 mL of THF, and then 0.9 mL of 1M $Na_2CO_3$ was added thereto. 144 mg of 4-(1-adamantyl)benzenesulfonyl chloride was slowly added dropwise at 0 °C and stirred at a room temperature for 2 hours. After the mixture was washed twice with 2 mL of diethyl ether, an aqueous layer was adjusted to pH 2 with 3M HCl. After the aqueous layer was extracted twice with 2 mL of ethyl acetate, it was dried using $MgSO_4$ and concentrated under reduced pressure to obtain 90 mg of 1-((4-((3r,5r,7r)-adamantan-1-yl)phenyl)sulfonyl)piperidine-2-carboxylic acid as an intermediate. The intermediate was used in the next reaction without any additional purification process.

50 mg of 1-((4-((3r,5r,7r)-adamantan-1-yl)phenyl)sulfonyl)piperidine-2-carboxylic acid was dissolved in 1 mL of THF along with 15 μL of NMO (N-methylmorpholine-N-oxide) and stirred at 0 °C. 13 μL of ethyl chloroformate was slowly added dropwise and stirred at the same temperature for 2 hours. After solids were filtered from the reaction solution and the obtained filtrate was stored separately, 12.9 mg of hydroxylamine hydrochloride and 26 μL of triethylamine were added dropwise to a solution dissolved in 1 mL of DMF. After stirring at a room temperature for 2 hours, the DMF was removed by concentration under reduced pressure. After the layers were separated by adding ethyl acetate and purified water, the aqueous layer was extracted twice more using ethyl acetate. After the organic layer was collected and concentrated under reduced pressure, the obtained solids were purified by a column chromatography (5 % MeOH in DCM) to produce 30 mg of 1-((4-((3r,5r,7r)-adamantan-1-yl)phenyl)sulfonyl)-N-hydroxypiperidine-2-carboxamide.
$^1$H-NMR (500 MHz, DMSO-*d*$_6$): δ 10.61 (s, 1H), 8.81 (s, 1H), 7.70 (m, 2H), 7.55 (m, 2H), 4.33 (m, 1H), 3.59 (m, 1H), 3.45 (m, 1H), 2.07 (s, 3H), 1.89 (m, 7H), 1.77 (m, 7H), 1.52 (m, 1H), 1.43 (m, 2H), 1.19 (m, 1H)

**(16) T-24** Synthesis of N-hydroxy-1-((4-propylphenyl)sulfonyl)piperidine-2-carboxamide

500 mg of pipecolinic acid was well stirred with 8 mL of THF, and then 8.9 mL of 1M $Na_2CO_3$ was added thereto. 831 μL of 4-n-propylbenzenesulfonyl chloride was slowly added dropwise at 0 °C and stirred at a room temperature for 2 hours. After the mixture was washed twice with 10 mL of diethyl ether, an aqueous layer was adjusted to pH 2 with 3M HCl. After the aqueous layer was extracted twice with 20 mL of ethyl acetate, it was dried using $MgSO_4$ and concentrated under reduced pressure to obtain 888 mg of 1-((4-propylphenyl)sulfonyl)piperidine-2-carboxylic acid as an intermediate. The intermediate was used in the next reaction without any additional purification process.

700 mg of 1-((4-propylphenyl)sulfonyl)piperidine-2-carboxylic acid was dissolved in 6 mL of THF along with 272 μL of NMO (N-methylmorpholine-N-oxide) and stirred at 0 °C. 236 μL of ethyl chloroformate was slowly added dropwise and stirred at the same temperature for 2 hours. After solids were filtered from the reaction solution and the obtained filtrate

was stored separately, 234 mg of hydroxylamine hydrochloride and 470 μL of triethylamine were added dropwise to a solution dissolved in 4 mL of DMF. After stirring at a room temperature for 2 hours, the DMF was removed by concentration under reduced pressure. After the layers were separated by adding ethyl acetate and purified water, the aqueous layer was extracted twice more using ethyl acetate. After the organic layer was collected and concentrated under reduced pressure, the obtained solids were purified by a column chromatography (5 % MeOH in DCM) to produce 0.16 g of N-hydroxy-1-((4-propylphenyl)sulfonyl)piperidine-2-carboxamide.
$^1$H-NMR (500 MHz, DMSO-$d_6$): δ 10.61 (s, 1H), 8.79 (s, 1H), 7.65 (m, 2H), 7.38 (m, 2H), 4.33 (m, 1H), 3.61 (m, 1H), 3.45 (m, 1H), 2.43 (t, 2H, *J*=7.5Hz), 1.76 (m, 1H), 1.59-1.38 (m, 6H), 1.16 (m, 1H), 0.90 (t, 3H, *J*=7.5Hz)

**(17) T-25** Synthesis of 1-((4-acetamidophenyl)sulfonyl)-N-hydroxypiperidine-2-carboxamide

500 mg of pipecolinic acid was well stirred with 8 mL of THF, and then 8.9 mL of 1M $Na_2CO_3$ was added thereto. 1.09 g of N-acetylsulfanilyl chloride was slowly added dropwise at 0 °C and stirred at a room temperature for 2 hours. After the mixture was washed twice with 10 mL of diethyl ether, an aqueous layer was adjusted to pH 2 with 3M HCl. After the aqueous layer was extracted twice with 20 mL of ethyl acetate, it was dried using $MgSO_4$ and concentrated under reduced pressure to obtain 935 mg of 1-((4-acetamidophenyl)sulfonyl)piperidine-2-carboxylic acid as an intermediate. The intermediate was used in the next reaction without any additional purification process.

800 mg of 1-((4-acetamidophenyl)sulfonyl)piperidine-2-carboxylic acid was dissolved in 6 mL of THF along with 296 μL of NMO (N-methylmorpholine-N-oxide) and stirred at 0 °C. 258 μL of ethyl chloroformate was slowly added dropwise and stirred at the same temperature for 2 hours. After solids were filtered from the reaction solution and the obtained filtrate was stored separately, 256 mg of hydroxylamine hydrochloride and 512 μL of triethylamine were added dropwise to a solution dissolved in 4 mL of DMF. After stirring at a room temperature for 2 hours, the DMF was removed by concentration under reduced pressure. After the layers were separated by adding ethyl acetate and purified water, the aqueous layer was extracted twice more using ethyl acetate. After the organic layer was collected and concentrated under reduced pressure, the obtained solids were purified by a column chromatography (5 % MeOH in DCM) to produce 0.18 g of 1-((4-acetamidophenyl)sulfonyl)-N-hydroxypiperidine-2-carboxamide.
$^1$H-NMR (500 MHz, DMSO-$d_6$): δ 10.60 (s, 1H), 10.32 (s, 1H), 8.78 (s, 1H), 7.74 (m, 2H), 7.68 (m, 2H), 4.30 (m, 1H), 3.61 (m, 1H), 3.45 (m, 1H), 2.09 (s, 3H), 1.76 (m, 1H), 1.51 (m, 1H), 1.39 (m, 3H), 1.16 (m, 1H)

**(18) T-28** Synthesis of 1-((4-(tert-butyl)phenyl)sulfonyl)-N-hydroxypyrrolidine-2-carboxamide

500 mg of proline was well stirred with 9 mL of THF, and then 10 mL of 1M $Na_2CO_3$ was added thereto. 1.21 g of 4-tert-butylbenzenesulfonyl chloride was slowly added dropwise at 0 °C and stirred at a room temperature for 2 hours. After the mixture was washed twice with 10 mL of diethyl ether, an aqueous layer was adjusted to pH 2 with 3M HCl. After the aqueous layer was extracted twice with 20 mL of ethyl acetate, it was dried using $MgSO_4$ and concentrated under reduced pressure to obtain 860 mg of ((4-(tert-butyl)phenyl)sulfonyl)proline as an intermediate. The intermediate was used in the next reaction without any additional purification process.

700 mg of ((4-(tert-butyl)phenyl)sulfonyl)proline was dissolved in 5 mL of THF along with 272 μL of NMO (N-methylmorpholine-N-oxide) and stirred at 0 °C. 236 μL of ethyl chloroformate was slowly added dropwise and stirred at the same temperature for 2 hours. After solids were filtered from the reaction solution and the obtained filtrate was stored separately, 234 mg of hydroxylamine hydrochloride and 470 μL of triethylamine were added dropwise to a solution dissolved in 4 mL of DMF. After stirring at a room temperature for 2 hours, the DMF was removed by concentration under reduced pressure. After the layers were separated by adding ethyl acetate and purified water, the aqueous layer was extracted twice more using ethyl acetate. After the organic layer was collected and concentrated under reduced pressure, the obtained solids were purified by a column chromatography (5 % MeOH in DCM) to produce 546 mg of 1-((4-(tert-butyl) phenyl)sulfonyl)-N-hydroxypyrrolidine-2-carboxamide.
$^1$H-NMR (500 MHz, DMSO-$d_6$): δ 10.66 (s, 1H), 8.94 (s, 1H), 7.78 (m, 2H), 7.64 (m, 2H), 3.91 (m, 1H), 3.30 (m, 1H), 3.13 (m, 1H), 1.88 (m, 1H) 1.75 (m, 1H) 1.66 (m, 1H), 1.46 (m, 1H), 1.31 (s, 9H)

**(19) T-29** Synthesis of 1-((4-butoxyphenyl)sulfonyl)-N-hydroxypyrrolidine-2-carboxamide

500 mg of proline was well stirred with 9 mL of THF, and then 10 mL of 1M $Na_2CO_3$ was added thereto. 1.30 g of 4-butoxybenzenesulfonyl chloride was slowly added dropwise at 0 °C and stirred at a room temperature for 2 hours. After the mixture was washed twice with 10 mL of diethyl ether, an aqueous layer was adjusted to pH 2 with 3M HCl. After the aqueous layer was extracted twice with 20 mL of ethyl acetate, it was dried using $MgSO_4$ and concentrated under reduced pressure to obtain 1.2 mg of ((4-butoxyphenyl)sulfonyl)proline as an intermediate. The intermediate was used in the next reaction without any additional purification process.

1 g of ((4-butoxyphenyl)sulfonyl)proline was dissolved in 7 mL of THF along with 369 μL of NMO (N-methyl-

morpholine-N-oxide) and stirred at 0 °C. 321 μL of ethyl chloroformate was slowly added dropwise and stirred at the same temperature for 2 hours. After solids were filtered from the reaction solution and the obtained filtrate was stored separately, 318 mg of hydroxylamine hydrochloride and 639 μL of triethylamine were added dropwise to a solution dissolved in 4 mL of DMF. After stirring at a room temperature for 2 hours, the DMF was removed by concentration under reduced pressure. After the layers were separated by adding ethyl acetate and purified water, the aqueous layer was extracted twice more using ethyl acetate. After the organic layer was collected and concentrated under reduced pressure, the obtained solids were purified by a column chromatography (5 % MeOH in DCM) to produce 370 mg of 1-((4-butoxyphenyl)sulfonyl)-N-hydroxypyrrolidine-2-carboxamide.
$^1$H-NMR (500 MHz, DMSO-$d_6$): δ 10.65 (s, 1H), 8.93 (s, 1H), 7.76 (m, 2H), 7.12 (m, 2H), 4.07 (t, 2H, *J*=6.5Hz), 3.88 (m, 1H), 3.39 (m, 1H), 3.11 (m, 1H), 1.83 (m, 1H), 1.74 (m, 3H), 1.63 (m, 1H), 1.45 (m, 3H), 0.94 (t, 3H, *J*=8.5Hz)

**(20) T-32** Synthesis of 1-((4-((3r,5r,7r)-adamantan-1-yl)phenyl)sulfonyl)-N-hydroxypyrrolidine-2-carboxamide

50 mg of proline was well stirred with 0.9 mL of THF, and then 1 mL of 1M $Na_2CO_3$ was added thereto. 162 mg of 4-(1-adamantyl)benzenesulfonyl chloride was slowly added dropwise at 0 °C and stirred at a room temperature for 2 hours. After the mixture was washed twice with 2 mL of diethyl ether, an aqueous layer was adjusted to pH 2 with 3M HCl. After the aqueous layer was extracted twice with 2 mL of ethyl acetate, it was dried using $MgSO_4$ and concentrated under reduced pressure to obtain 171 mg of ((4-((3r,5r,7r)-adamantan-1-yl)phenyl)sulfonyl)proline as an intermediate. The intermediate was used in the next reaction without any additional purification process.

130 mg of ((4-((3r,5r,7r)-adamantan-1-yl)phenyl)sulfonyl)proline was dissolved in 2 mL of THF along with 40 μL of NMO (N-methylmorpholine-N-oxide) and stirred at 0 °C. 35 μL of ethyl chloroformate was slowly added dropwise and stirred at the same temperature for 2 hours. After solids were filtered from the reaction solution and the obtained filtrate was stored separately, 35 mg of hydroxylamine hydrochloride and 70 μL of triethylamine were added dropwise to a solution dissolved in 1 mL of DMF. After stirring at a room temperature for 2 hours, the DMF was removed by concentration under reduced pressure. After the layers were separated by adding ethyl acetate and purified water, the aqueous layer was extracted twice more using ethyl acetate. After the organic layer was collected and concentrated under reduced pressure, the obtained solids were purified by a column chromatography (5 % MeOH in DCM) to produce 90 mg of 1-((4-((3r,5r,7r)-adamantan-1-yl)phenyl)sulfonyl)-N-hydroxypyrrolidine-2-carboxamide.
$^1$H-NMR (500 MHz, DMSO-$d_6$): δ 10.66 (s, 1H), 8.95 (s, 1H), 7.77 (m, 2H), 7.62 (m, 2H), 3.91 (m, 1H), 3.39 (m, 1H), 3.12 (m, 1H), 2.15 (s, 3H), 1.89-1.75 (m, 13H), 1.65 (m, 1H), 1.50 (m, 1H), 1.16 (m, 1H)

**(21) T-33** Synthesis of N-hydroxy-1-((4-propylphenyl)sulfonyl)pyrrolidine-2-carboxamide

500 mg of proline was well stirred with 9 mL of THF, and then 10 mL of 1M $Na_2CO_3$ was added thereto. 932 μL of 4-n-propylbenzenesulfonyl chloride was slowly added dropwise at 0 °C and stirred at a room temperature for 2 hours. After the mixture was washed twice with 10 mL of diethyl ether, an aqueous layer was adjusted to pH 2 with 3M HCl. After the aqueous layer was extracted twice with 20 mL of ethyl acetate, it was dried using $MgSO_4$ and concentrated under reduced pressure to obtain 1 g of ((4-propylphenyl)sulfonyl)proline as an intermediate. The intermediate was used in the next reaction without any additional purification process.

900 mg of ((4-propylphenyl)sulfonyl)proline was dissolved in 6 mL of THF along with 366 μL of NMO (N-methylmorpholine-N-oxide) and stirred at 0 °C. 318 μL of ethyl chloroformate was slowly added dropwise and stirred at the same temperature for 2 hours. After solids were filtered from the reaction solution and the obtained filtrate was stored separately, 315 mg of hydroxylamine hydrochloride and 633 μL of triethylamine were added dropwise to a solution dissolved in 4 mL of DMF. After stirring at a room temperature for 2 hours, the DMF was removed by concentration under reduced pressure. After the layers were separated by adding ethyl acetate and purified water, the aqueous layer was extracted twice more using ethyl acetate. After the organic layer was collected and concentrated under reduced pressure, the obtained solids were purified by a column chromatography (5 % MeOH in DCM) to produce 665 mg of N-hydroxy-1-((4-propylphenyl)sulfonyl)pyrrolidine-2-carboxamide.
$^1$H-NMR (500 MHz, DMSO-$d_6$): δ 10.66 (s, 1H), 8.94 (s, 1H), 7.76 (m, 2H), 7.46 (m, 2H), 3.90 (m, 1H), 3.38 (m, 1H), 3.14 (m, 1H), 2.66 (t, 2H, *J*=9Hz), 1.83 (m, 1H), 1.75 (m, 1H), 1.65 (m, 2H), 1.44 (m, 2H), 0.89 (t, 3H, *J*=7.5Hz)

**(22) T-34** Synthesis of 1-((4-acetylphenyl)sulfonyl)-N-hydroxypyrrolidine-2-carboxamide

500 mg of proline was well stirred with 9 mL of THF, and then 10 mL of 1M $Na_2CO_3$ was added thereto. 1.14 g of 4-acetylbenzenesulfonyl chloride was slowly added dropwise at 0 °C and stirred at a room temperature for 2 hours. After the mixture was washed twice with 10 mL of diethyl ether, an aqueous layer was adjusted to pH 2 with 3M HCl. After the aqueous layer was extracted twice with 20 mL of ethyl acetate, it was dried using $MgSO_4$ and concentrated under reduced pressure to obtain 958 mg of ((4-acetylphenyl)sulfonyl)proline as an intermediate. The intermediate was used in the next

reaction without any additional purification process.

800 mg of ((4-acetylphenyl)sulfonyl)proline was dissolved in 6 mL of THF along with 325 μL of NMO (N-methylmorpholine-N-oxide) and stirred at 0 °C. 283 μL of ethyl chloroformate was slowly added dropwise and stirred at the same temperature for 2 hours. After solids were filtered from the reaction solution and the obtained filtrate was stored separately, 280 mg of hydroxylamine hydrochloride and 563 μL of triethylamine were added dropwise to a solution dissolved in 4 mL of DMF. After stirring at a room temperature for 2 hours, the DMF was removed by concentration under reduced pressure. After the layers were separated by adding ethyl acetate and purified water, the aqueous layer was extracted twice more using ethyl acetate. After the organic layer was collected and concentrated under reduced pressure, the obtained solids were purified by a column chromatography (5 % MeOH in DCM) to produce 110 mg of 1-((4-acetylphenyl)sulfonyl)-N-hydroxypyrrolidine-2-carboxamide.

$^1$H-NMR (500 MHz, DMSO-$d_6$): δ 10.72 (s, 1H), 8.97 (s, 1H), 8.15 (m, 2H), 7.97 (m, 2H), 3.95 (m, 1H), 3.43 (m, 1H), 3.13 (m, 1H), 2.65 (s, 3H), 1.86 (m, 1H), 1.72 (m, 2H), 1.48 (m, 1H)

**(23) T-35** Synthesis of N-hydroxy-1-((4-(1-(hydroxyimino)ethyl)phenyl)sulfonyl)piperidine-2-carboxamide

1 g of pipecolinic acid was well stirred with 16 mL of THF, and then 17.8 mL of 1M $Na_2CO_3$ was added thereto. 2.04 g of 4-acetylbenzenesulfonyl chloride was slowly added dropwise at 0 °C and stirred at a room temperature for 2 hours. After the mixture was washed twice with 20 mL of diethyl ether, an aqueous layer was adjusted to pH 2 with 3M HCl. After the aqueous layer was extracted twice with 40 mL of ethyl acetate, it was dried using $MgSO_4$ and concentrated under reduced pressure to obtain 1.21 g of 1-((4-(1-(hydroxyamino)ethyl)phenyl)sulfonyl)piperidine-2-carboxylic acid as an intermediate. The intermediate was used in the next reaction without any additional purification process.

1 g of 1-((4-(1-(hydroxyamino)ethyl)phenyl)sulfonyl)piperidine-2-carboxylic acid was dissolved in 10 mL of THF along with 388 μL of NMO (N-methylmorpholine-N-oxide) and stirred at 0 °C. 338 μL of ethyl chloroformate was slowly added dropwise and stirred at the same temperature for 2 hours. After solids were filtered from the reaction solution and the obtained filtrate was stored separately, 446 mg of hydroxylamine hydrochloride and 895 μL of triethylamine were added dropwise to a solution dissolved in 6 mL of DMF. After stirring at a room temperature for 2 hours, the DMF was removed by concentration under reduced pressure. After the layers were separated by adding ethyl acetate and purified water, the aqueous layer was extracted twice more using ethyl acetate. After the organic layer was collected and concentrated under reduced pressure, the obtained solids were purified by a column chromatography (5 % MeOH in DCM) to produce 0.18 g of N-hydroxy-1-((4-(1-(hydroxyimino)ethyl)phenyl)sulfonyl)piperidine-2-carboxamide.

$^1$H-NMR (500 MHz, DMSO-$d_6$): δ 11.55 (s, 1H), 10.63 (s, 1H), 8.79 (s, 1H), 7.82 (m, 2H), 7.75 (m, 2H), 4.35 (m, 1H), 3.63 (m, 1H), 3.47 (m, 1H), 3.32 (s, 3H), 1.74 (m, 1H), 1.54 (m, 1H), 1.45 (m, 3H), 1.20 (m, 1H)

**(24) T-36** Synthesis of N-hydroxy-4-methyl-2-(phenylsulfonamido)pentanamide

500 mg of leucine was well stirred with 8 mL of THF, and then 8.8 mL of 1M $Na_2CO_3$ was added thereto. 584 μL of benzenesulfonyl chloride was slowly added dropwise at 0 °C and stirred at a room temperature for 1 hour. After the mixture was washed twice with 10 mL of diethyl ether, an aqueous layer was adjusted to pH 3 with 3M HCl. After the aqueous layer was extracted with 20 mL of ethyl acetate, it was dried using $MgSO_4$ and concentrated under reduced pressure to obtain 444 mg of (phenylsulfonyl)leucine as an intermediate. The intermediate was used in the next reaction without any additional purification process.

400 mg of (phenylsulfonyl)leucine was dissolved in 5 mL of THF along with 178 μL of NMO (N-methylmorpholine-N-oxide) and stirred at 0 °C. 155 μL of ethyl chloroformate was slowly added dropwise and stirred at the same temperature for 30 minutes. After solids were filtered from the reaction solution and the obtained filtrate was stored separately, 154 mg of hydroxylamine hydrochloride and 308 μL of triethylamine were added dropwise to a solution dissolved in 2 mL of DMF. After stirring at a room temperature for 30 minutes, the DMF was removed by concentration under reduced pressure. After the layers were separated by adding ethyl acetate and purified water, the aqueous layer was extracted twice more using ethyl acetate. After the organic layer was collected and concentrated under reduced pressure, the obtained solids were purified by a column chromatography (hexane: ethyl acetate = 1:1) to produce 0.11 g of N-hydroxy-4-methyl-2-(phenylsulfonamido)pentanamide.

$^1$H-NMR (500 MHz, DMSO-$d_6$): δ 10.65 (s, 1H), 8.81 (s, 1H), 8.00 (m, 1H), 7.77 (m, 2H), 7.57 (m, 1H), 7.55 (m, 2H), 3.48 (m, 1H), 1.43 (m, 1H), 1.35-1.20 (m, 2H), 0.72 (d, 3H, *J*=6.5Hz), 0.60 (d, 3H, *J*=7.0Hz)

**(25) T-40** Synthesis of 1-((4-acetamidophenyl)sulfonyl)-N-hydroxypyrrolidine-2-carboxamide

1 g of proline was well stirred with 18 mL of THF, and then 20 mL of 1M $Na_2CO_3$ was added thereto. 2.44 g of N-acetylsulfanilyl chloride was slowly added dropwise at 0 °C and stirred at a room temperature for 2 hours. After the mixture was washed twice with 20 mL of diethyl ether, an aqueous layer was adjusted to pH 2 with 3M HCl. After the aqueous layer

was extracted twice with 40 mL of ethyl acetate, it was dried using $MgSO_4$ and concentrated under reduced pressure to obtain 483 mg of ((4-acetamidophenyl)sulfonyl)proline as an intermediate. The intermediate was used in the next reaction without any additional purification process.

450 mg of ((4-acetamidophenyl)sulfonyl)proline was dissolved in 6 mL of THF along with 174 μL of NMO (N-methylmorpholine-N-oxide) and stirred at 0 °C. 152 μL of ethyl chloroformate was slowly added dropwise and stirred at the same temperature for 2 hours. After solids were filtered from the reaction solution and the obtained filtrate was stored separately, 200 mg of hydroxylamine hydrochloride and 401 μL of triethylamine were added dropwise to a solution dissolved in 1.5 mL of DMF. After stirring at a room temperature for 2 hours, the DMF was removed by concentration under reduced pressure. After the layers were separated by adding ethyl acetate and purified water, the aqueous layer was extracted twice more using ethyl acetate. After the organic layer was collected and concentrated under reduced pressure, the obtained solids were purified by a column chromatography (5 % MeOH in DCM) to produce 120 mg of 1-((4-acetamidophenyl)sulfonyl)-N-hydroxypyrrolidine-2-carboxamide.

$^1$H-NMR (500 MHz, DMSO-$d_6$): δ 10.64 (s, 1H), 10.38 (s, 1H), 8.91 (s, 1H), 7.79 (m, 4H), 3.90 (m, 1H), 3.41 (m, 1H), 3.11 (m, 1H), 2.09 (s, 3H), 1.82 (m, 1H), 1.70 (m, 2H), 1.42 (m, 1H)

## Experiment Example

The MMPs inhibition efficacy of the new compound was confirmed through experiments as follows.

### 1. Experimental design

Enzyme activity (%) at the same concentration was compared with treatment with 0.5 μM MMP-1 activity and 50 nM MMP-2 and MMP-9 activity (n=2). Here, enzyme activity (%) refers to the enzyme activity (%) of the experimental group when the untreated group is considered to have 100% enzyme activity.

### 2. Assessment of MMP activity

To evaluate the effect of the new compound on the enzyme activity, the color change was measured by following the provided protocol using a kit sold by Abchem as follows. Enzyme activity was obtained by comparing absorbance per minute with the untreated group.

- MMP1 Inhibitor Screening Assay Kit (ab139443)
- MMP2 Inhibitor Screening Assay Kit (ab139446)
- MMP9 Inhibitor Screening Assay Kit (ab139448)

### 3. MMP activity evaluation principle

As MMP activity increases, the substrate is decomposed and the OD412 level increases. Therefore, the change in OD412 level (OD value) per minute was measured and the value was obtained according to the enzyme activity calculation formula below. Here, OD412 means optical density or absorbance at 412 nm.

- Thiopeptide (Ac-PLG-[2-mercapto-4-methyl-pentanoyl]-LG-OC2H5) as a chromogenic substrate.
- The MMP cleavage site peptide bond was replaced by the thioester bond of the thiopeptide.
- Hydrolysis of this bond by MMP generates a sulfhydryl group, which reacts with DTNB [5,5'-dithiobis(2-nitrobenzoic acid), Ellman's reagent] to form 2-nitro-5-thiobenzoic acid. , which can be measured by its absorbance at 412 nm. ($\varepsilon$=13,600 $M^{-1}cm^{-1}$ above pH 6.0)

**<Enzyme activity calculation formula>'**

$$\text{New compound enzyme activity (\%)} = (V_{\text{new compound}}/V_{\text{untreated group}})*100$$

V = change in OD value per minute

The enzyme activity calculation formula may be expressed as follows.

<Inhibition effect calculation formula>

Inhibitory efficacy of new compound (%) = Enzyme activity of untreated group - Enzyme activity of new compound = 100 - New compound enzyme activity

**4. Experimental results**

The results of comparing single concentrations of 25 new compounds for MMPs enzyme activity and inhibitory efficacy were shown in Table 2 (enzyme activity, unit: %) and Table 3 (inhibitory activity, unit: %).

[Table 2]

| TM No. | MMP-1 (%) at 0.5 uM | MMP-2 (%) at 50 nM | MMP-9 (%) at 50 nM |
|---|---|---|---|
| Untreated group | 100.0 | 100.0 | 100.0 |
| T-01 | 34.2 | 81.6 | 79.1 |
| T-02 | 40.5 | 0.8 | 41.9 |
| T-04 | 51.4 | 1.5 | 0.0 |
| T-05 | 100.0 | 100.0 | 100.0 |
| T-08 | 100.0 | 100.0 | 100.0 |
| T-10 | 100.0 | 95.6 | 100.0 |
| T-11 | 100.0 | 82.7 | 100.0 |
| T-12 | 52.5 | 0.0 | 70.6 |
| T-13 | 100.0 | 100.0 | 100.0 |
| T-14 | 100.0 | 0.0 | 73.5 |
| T-15 | 100.0 | 100.0 | 100.0 |
| T-18 | 100.0 | 99.9 | 100.0 |
| T-20 | 100.0 | 97.8 | 100.0 |
| T-21 | 9.2 | 81.1 | 100.0 |
| T-23 | 100.0 | 100.0 | 100.0 |
| T-24 | 78.4 | 57.7 | 99.0 |
| T-25 | 100.0 | 100.0 | 100.0 |
| T-28 | 100.0 | 100.0 | 100.0 |
| T-29 | 100.0 | 18.9 | 50.2 |
| T-32 | 100.0 | 100.0 | 100.0 |
| T-33 | 100.0 | 90.5 | 100.0 |
| T-34 | 100.0 | 100.0 | 100.0 |
| T-35 | 9.7 | 24.6 | 19.9 |
| T-36 | 98.2 | 100.0 | 97.4 |
| T-40 | 100.0 | 100.0 | 100.0 |

[Table 3]

| TM No. | MMP-1 (%) at 0.5 uM | MMP-2 (%) at 50 nM | MMP-9 (%) at 50 nM |
|---|---|---|---|
| Untreated group | 0.0 | 0.0 | 0.0 |
| T-01 | 65.8 | 18.4 | 20.9 |

(continued)

| TM No. | MMP-1 (%) at 0.5 uM | MMP-2 (%) at 50 nM | MMP-9 (%) at 50 nM |
|---|---|---|---|
| T-02 | 59.5 | 99.2 | 58.1 |
| T-04 | 48.6 | 98.5 | 100.0 |
| T-05 | 0.0 | 0.0 | 0.0 |
| T-08 | 0.0 | 0.0 | 0.0 |
| T-10 | 0.0 | 4.4 | 0.0 |
| T-11 | 0.0 | 17.3 | 0.0 |
| T-12 | 47.5 | 100.0 | 29.4 |
| T-13 | 0.0 | 0.0 | 0.0 |
| T-14 | 0.0 | 100.0 | 26.5 |
| T-15 | 0.0 | 0.0 | 0.0 |
| T-18 | 0.0 | 0.1 | 0.0 |
| T-20 | 0.0 | 2.2 | 0.0 |
| T-21 | 90.8 | 18.9 | 0.0 |
| T-23 | 0.0 | 0.0 | 0.0 |
| T-24 | 21.6 | 42.3 | 1.0 |
| T-25 | 0.0 | 0.0 | 0.0 |
| T-28 | 0.0 | 0.0 | 0.0 |
| T-29 | 0.0 | 81.1 | 49.8 |
| T-32 | 0.0 | 0.0 | 0.0 |
| T-33 | 0.0 | 9.5 | 0.0 |
| T-34 | 0.0 | 0.0 | 0.0 |
| T-35 | 90.3 | 75.4 | 80.1 |
| T-36 | 1.8 | 0.0 | 2.6 |
| T-40 | 0.0 | 0.0 | 0.0 |

Tables 2 and 3 above show the results of experiments with 3 different SETs. For example, in the case of T-01, when treated with MMP-1 at 0.5 uM, 34.2% (from 100%) of the enzyme activity of MMP-1 remained. am. For MMP-2, the enzyme activity was 81.6% (from 100%) when treated with 50 nM of T-01, and for MMP-9, the enzyme activity was 79.1% (from 100%) when treated with 50 nM of T-01. .

As can be seen from the experimental results, in most cases, MMP-2 and MMP-9 are inhibited even at the 50 nM level, whereas MMP-1 was confirmed to be inhibited at 0.5 uM, which is 10 times higher than this.

In addition, each compound has a different degree of inhibition of the activity of three types of enzymes, namely MMP-1, MMP-2, and MMP-9, and this varies depending on the structural characteristics of MMP-1, MMP-2, and MMP-9. It can be assumed that this is a result of the difference in the combination of .

In other words, the reason for the difference in the activity inhibition strength of T-01 to T-40 against MMPs is the result of slightly different structures. Depending on the structure, the enzyme binding ability is different and the activity inhibition effect is also different. It was confirmed that occurred.

Table 4 below shows the enzyme activity (%) when treated at high concentration, and was treated according to the following conditions.

MMP-1 (%) : T-05~20 at 10 uM, T-23~34 at 5 uM
MMP-2 (%) : T-05~20 at 10 uM, T-23~34 at 0.5 uM
MMP-9 (%) : T-05~20 not tested, T-23~34 at 0.5 uM

[Table 4]

| TM No. | MMP-1 (%) | MMP-2 (%) | MMP-9 (%) |
|---|---|---|---|
| Untreated group | 100 | 100 | 100 |
| T-05 | 91.7 | 84.2 | n.t |
| T-08 | 87.2 | 100 | n.t |
| T-10 | 100 | 84.5 | n.t |
| T-11 | 57.1 | 6.2 | n.t |
| T-13 | 100 | 28.1 | n.t |
| T-15 | 100 | 65.2 | n.t |
| T-18 | 100 | 100 | n.t |
| T-20 | 100 | 40.1 | n.t |
| T-23 | 100 | 100 | 100 |
| T-25 | 85.8 | 61.7 | 79.4 |
| T-28 | 100 | 100 | 100 |
| T-32 | 100 | 92.4 | 88.1 |
| T-33 | 73.9 | 73.7 | 77.8 |
| T-34 | 41.8 | 62.2 | 69.9 |

Table 5 below shows the inhibitory efficacy (%) when treated at high concentration, and was treated according to the following conditions.

MMP-1 (%) : T-05~20 at 10 uM, T-23~34 at 5 uM
MMP-2 (%) : T-05~20 at 10 uM, T-23~34 at 0.5 uM
MMP-9 (%) : T-05~20 not tested, T-23~34 at 0.5 uM

[Table 5]

| TM No. | MMP-1 (%) | MMP-2 (%) | MMP-9 (%) |
|---|---|---|---|
| Untreated group | 0.0 | 0.0 | 0.0 |
| T-05 | 8.3 | 15.8 | n.t |
| T-08 | 12.8 | 0.0 | n.t |
| T-10 | 0.0 | 15.5 | n.t |
| T-11 | 42.9 | 93.8 | n.t |
| T-13 | 0.0 | 71.9 | n.t |
| T-15 | 0.0 | 34.8 | n.t |
| T-18 | 0.0 | 0.0 | n.t |
| T-20 | 0.0 | 59.9 | n.t |
| T-23 | 0.0 | 0.0 | 0.0 |
| T-25 | 14.2 | 38.3 | 20.6 |
| T-28 | 0.0 | 0.0 | 0.0 |
| T-32 | 0.0 | 7.6 | 11.9 |
| T-33 | 26.1 | 26.3 | 22.2 |
| T-34 | 58.2 | 37.8 | 30.1 |

**Embodiments**

Embodiment 1: A compound represented by the following formula 1, an isomer thereof, a pharmaceutically acceptable salt thereof, a hydrate thereof, or a solvate thereof:

[Formula 1]

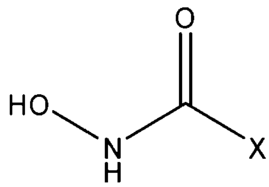

In Formula 1,
X is represented by any one of the following formulas 1-1 to 1-5,

[Formula 1-1]

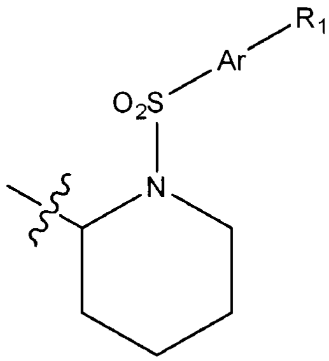

[Formula 1-2]

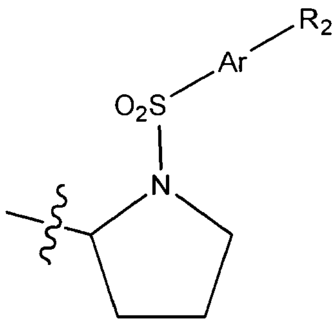

[Formula 1-3]

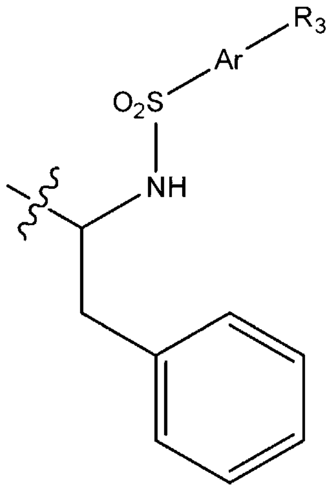

[Formula 1-4]

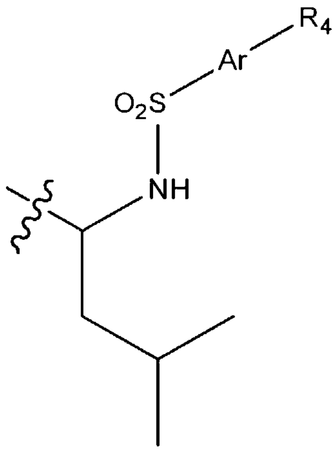

[Formula 1-5]

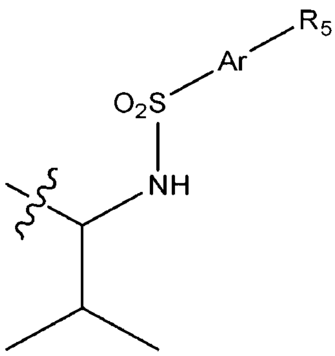

In Formula 1-1,

Ar is a phenyl unsubstituted or substituted with 1 to 3 $R_1$,
$R_1$ is a hydrogen, a phenyl group, a $C_1$-$C_4$ alkoxy group, a $C_1$-$C_3$ alkyl group, a fluoro group, an adamantane group, a acetylamino group, or a hydroxyiminoethyl group, when there are 2 or 3 $R_1$ residues, these $R_1$ residues are identical with each other,
In Formula 1-2,
Ar is a phenyl substituted with one $R_2$,
$R_2$ is a $C_1$-$C_4$ alkyl group, a $C_1$-$C_4$ alkoxy group, an adamantane group, a acetyl group, or a acetylamino group,

In Formula 1-3,
Ar is a phenyl substituted with 1-3 $R_3$ or an unsubstituted naphthalene,
$R_3$ is a hydrogen or a $C_1$-$C_4$ alkyl group, when there are 2 or 3 $R_3$ residues, these $R_3$ residues are identical with each other, preferably $R_3$ is a $C_1$-$C_4$ alkyl group,
In Formula 1-4,
Ar is a phenyl unsubstituted or substituted with 1 to 3 $R_4$,
$R_4$ is a hydrogen, a phenyl group, a $C_1$-$C_4$ alkyl group, or a $C_1$-$C_4$ alkoxy group, when there are 2 or 3 $R_4$ residues, these $R_4$ residues are identical with each other,
In Formula 1-5,
Ar is a phenyl substituted with 1 to 3 $R_5$,
$R_5$ is a $C_1$-$C_4$ alkyl group, when there are 2 or 3 $R_5$ residues, these $R_5$ residues are identical with each other.

Embodiment 2: The compound represented by formula 1 according to Embodiment 1, comprising any one of the following compounds:

N-hydroxy-1-(phenylsulfonyl)piperidine-2-carboxamide,
1-([1,1'-biphenyl]-4-ylsulfonyl)-N-hydroxypiperidine-2-carboxamide,
1-((4-butoxyphenyl)sulfonyl)-N-hydroxypiperidine-2-carboxamide,
N-hydroxy-1-(mesitylsulfonyl)piperidine-2-carboxamide,
2-((4-(tert-butyl)phenyl)sulfonamido)-N-hydroxy-3-phenylpropanamide,
N-hydroxy-3-phenyl-2-((2,4,6-trimethylphenyl)sulfonamido)propanamide,
N-hydroxy-2-(naphthalene-2-sulfonamido)-3-phenylpropanamide,
2-([1,1'-biphenyl]-4-sulfonamido)-N-hydroxy-4-methylpentanamide,
2-((4-(tert-butyl)phenyl)sulfonamido)-N-hydroxy-4-methylpentanamide,
2-((4-butoxyphenyl)sulfonamido)-N-hydroxy-4-methylpentanamide,
N-hydroxy-4-methyl-2-((2,4,6-trimethylphenyl)sulfonamido)pentanamide,
2-((4-(tert-butyl)phenyl)sulfonamido)-N-hydroxy-3-methylbutanamide,
N-hydroxy-3-methyl-2-((2,4,6-trimethylphenyl)sulfonamido)butanamide,
1-((4-fluorophenyl)sulfonyl)-N-hydroxypiperidine-2-carboxamide,
1-((4-((3r,5r,7r)-adamantan-1-yl)phenyl)sulfonyl)-N-hydroxypiperidine-2-carboxamide,
N-hydroxy-1-((4-propylphenyl)sulfonyl)piperidine-2-carboxamide,
1-((4-acetamidophenyl)sulfonyl)-N-hydroxypiperidine-2-carboxamide,
1-((4-(tert-butyl)phenyl)sulfonyl)-N-hydroxypyrrolidine-2-carboxamide,
1-((4-butoxyphenyl)sulfonyl)-N-hydroxypyrrolidine-2-carboxamide,
1-((4-((3r,5r,7r)-adamantan-1-yl)phenyl)sulfonyl)-N-hydroxypyrrolidine-2-carboxamide,
N-hydroxy-1-((4-propylphenyl)sulfonyl)pyrrolidine-2-carboxamide,
1-((4-acetylphenyl)sulfonyl)-N-hydroxypyrrolidine-2-carboxamide,
N-hydroxy-1-((4-(1-(hydroxyimino)ethyl)phenyl)sulfonyl)piperidine-2-carboxamide,
N-hydroxy-4-methyl-2-(phenylsulfonamido)pentanamide, and
1-((4-acetamidophenyl)sulfonyl)-N-hydroxypyrrolidine-2-carboxamide.

Embodiment 3: The compound represented by formula 1 according to claims 1 or 2, comprising the any one of the following compounds:

N-hydroxy-1-(phenylsulfonyl)piperidine-2-carboxamide,
1-([1,1'-biphenyl]-4-ylsulfonyl)-N-hydroxypiperidine-2-carboxamide,
1-((4-butoxyphenyl)sulfonyl)-N-hydroxypiperidine-2-carboxamide,
N-hydroxy-1-(mesitylsulfonyl)piperidine-2-carboxamide,
2-((4-(tert-butyl)phenyl)sulfonamido)-N-hydroxy-3-phenylpropanamide,
N-hydroxy-3-phenyl-2-((2,4,6-trimethylphenyl)sulfonamido)propanamide,
N-hydroxy-2-(naphthalene-2-sulfonamido)-3-phenylpropanamide,
2-([1,1'-biphenyl]-4-sulfonamido)-N-hydroxy-4-methylpentanamide,
2-((4-(tert-butyl)phenyl)sulfonamido)-N-hydroxy-4-methylpentanamide,
2-((4-butoxyphenyl)sulfonamido)-N-hydroxy-4-methylpentanamide,
N-hydroxy-4-methyl-2-((2,4,6-trimethylphenyl)sulfonamido)pentanamide,
N-hydroxy-3-methyl-2-((2,4,6-trimethylphenyl)sulfonamido)butanamide,
1-((4-fluorophenyl)sulfonyl)-N-hydroxypiperidine-2-carboxamide,
N-hydroxy-1-((4-propylphenyl)sulfonyl)piperidine-2-carboxamide,

1-((4-acetamidophenyl)sulfonyl)-N-hydroxypiperidine-2-carboxamide,
1-((4-butoxyphenyl)sulfonyl)-N-hydroxypyrrolidine-2-carboxamide,
1-((4-((3r,5r,7r)-adamantan-1-yl)phenyl)sulfonyl)-N-hydroxypyrrolidine-2-carboxamide,
N-hydroxy-1-((4-propylphenyl)sulfonyl)pyrrolidine-2-carboxamide,
1-((4-acetylphenyl)sulfonyl)-N-hydroxypyrrolidine-2-carboxamide,
N-hydroxy-1-((4-(1-(hydroxyimino)ethyl)phenyl)sulfonyl)piperidine-2-carboxamide, and
N-hydroxy-4-methyl-2-(phenylsulfonamido)pentanamide

Embodiment 4: The compound represented by formula 1 according to any one of Embodiments1-3, comprising the any one of the following compounds:

2-((4-(tert-butyl)phenyl)sulfonamido)-N-hydroxy-3-methylbutanamide,
1-((4-((3r,5r,7r)-adamantan-1-yl)phenyl)sulfonyl)-N-hydroxypiperidine-2-carboxamide,
1-((4-(tert-butyl)phenyl)sulfonyl)-N-hydroxypyrrolidine-2-carboxamide, and
1-((4-acetamidophenyl)sulfonyl)-N-hydroxypyrrolidine-2-carboxamide.

Embodiment 5: The compound represented by formula 1 according to any one of Embodiments 14, wherein the compound is derived from an amino acid.

Embodiment 6: A cosmetic composition comprising, as active ingredients, the compound represented by formula 1 according to any one of Embodiments 1 to 5, an isomer thereof, a pharmaceutically acceptable salt thereof, a hydrate thereof, or a solvate thereof.

Embodiment 7: The cosmetic composition according to Embodiment 6, wherein a content of the compound represented by formula 1, the isomer thereof, the pharmaceutically acceptable salt thereof, the hydrate thereof, or the solvate thereof is 0.01 μM to 100 mM based on the total volume of the composition.

Embodiment 8: A food composition comprising, as active ingredients, the compound represented by formula 1 according to any one of Embodiments 1 to 5, an isomer thereof, a pharmaceutically acceptable salt thereof, a hydrate thereof, or a solvate thereof.

Embodiment 9: The food composition according to Embodiment 8, wherein a content of the compound represented by formula 1, the isomer thereof, the pharmaceutically acceptable salt thereof, the hydrate thereof, or the solvate thereof is 0.01 μM to 100 mM based on the total volume of the composition.

Embodiment 10: A composition for inhibiting MMP-1 activity comprising, as active ingredients, the compound represented by formula 1 according to any one of Embodiments 1 to 5, an isomer thereof, a pharmaceutically acceptable salt thereof, a hydrate thereof, or a solvate thereof.

Embodiment 11: The composition for inhibiting the MMP-1 activity according to Embodiment 10, wherein a content of the compound represented by formula 1, the isomer thereof, the pharmaceutically acceptable salt thereof, the hydrate thereof, or the solvate thereof is 0.01 μM to 100 mM based on the total volume of the composition.

Embodiment 12: A composition for inhibiting MMP-2 activity comprising, as active ingredients, the compound represented by formula 1 according to any one of Embodiments 1 to 5, an isomer thereof, a pharmaceutically acceptable salt thereof, a hydrate thereof, or a solvate thereof.

Embodiment 13: The composition for inhibiting the MMP-2 activity according to Embodiment 12, wherein a content of the compound represented by formula 1, the isomer thereof, the pharmaceutically acceptable salt thereof, the hydrate thereof, or the solvate thereof is 0.01 μM to 100 mM based on the total volume of the composition.

Embodiment 14: A composition for inhibiting MMP-9 activity comprising, as active ingredients, the compound represented by formula 1 according to any one of Embodiments 1 to 5, an isomer thereof, a pharmaceutically acceptable salt thereof, a hydrate thereof, or a solvate thereof.

Embodiment15: The composition for inhibiting the MMP-9 activity according to Embodiment 14, wherein a content of the compound represented by formula 1, the isomer thereof, the pharmaceutically acceptable salt thereof, the hydrate thereof, or the solvate thereof is 0.01 μM to 100 mM based on the total volume of the composition.

Embodiment 16: A method for preparing the compound represented by formula 1 according to any one of Embodiments 1 to 5, comprising reacting an amino acid selected from pipecolinic acid, phenylalanine, leucine, valine and proline with any one selected from sulfonic acid, sulfonyl halide, sulfanilic acid, and sulfanilyl halide.

Embodiment 17: The method according to Embodiment 16, wherein the method further comprises obtaining the compound represented by formula 1 by reacting the intermediate obtained through the above reaction with a hydroxylamine(free form) or a hydroxylamine salt.

Embodiment 18: The method according to Embodiments 16 or 17, wherein the sulfonyl halide is a sulfonyl fluoride, a sulfonyl chloride, a sulfonyl bromide, or a sulfonyl iodide, and the sulfanilyl halide is a sulfanylyl fluoride, a sulfanylyl chloride, a sulfanylyl bromide, or a sulfanylyl iodide.

Embodiment 19: The method according to any one of Embodiments 16-18, wherein the sulfonyl chloride is any

one selected from a group of a benzenesulfonyl chloride, 4-biphenylsulfonyl chloride, 4-butoxybenzene-1-sulfonyl chloride, 2-mesitylenesulfonyl chloride, 4-tert-butylbenzenesulfonyl chloride, 2-naphthalenesulfonyl chloride, 4-fluorobenzenesulfonyl chloride, 4-(1-adamantyl)benzenesulfonyl chloride, 4-n-propylbenzenesulfonyl chloride, and 4-acetylbenzenesulfonyl chloride, and the sulfanylyl chloride is N-acetylsulfanylyl chloride.

## Claims

1. A compound represented by the following formula 1, an isomer thereof, a pharmaceutically acceptable salt thereof, a hydrate thereof, or a solvate thereof:

[Formula 1]

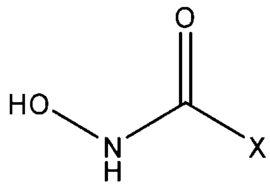

wherein in Formula 1,
X is represented by any one of the following formulas 1-1 to 1-5,

[Formula 1-1]

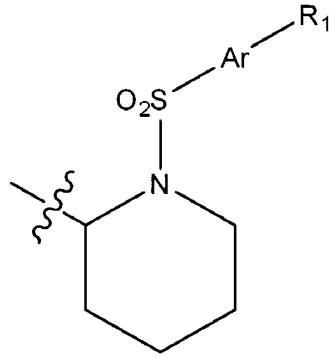

[Formula 1-2]

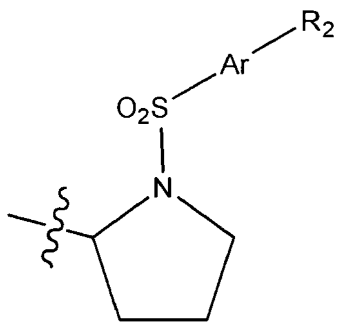

[Formula 1-3]

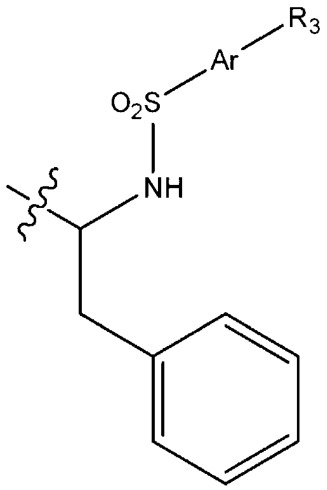

[Formula 1-4]

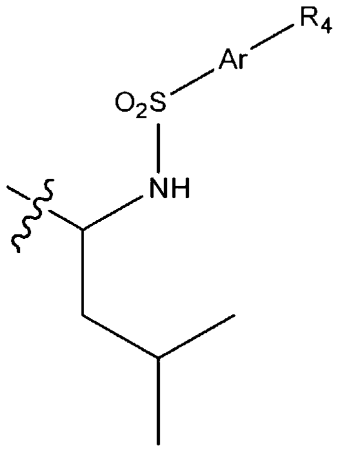

[Formula 1-5]

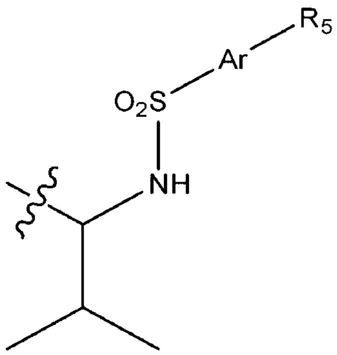

wherein in Formula 1-1,

Ar is a phenyl unsubstituted or substituted with 1 to 3 $R_1$,
$R_1$ is a hydrogen, a phenyl group, a $C_1$-$C_4$ alkoxy group, a $C_1$-$C_3$ alkyl group, a fluoro group, an adamantane group, a acetylamino group, or a hydroxyiminoethyl group, wherein when more than one $R_1$ is present, all $R_1$ residues are identical,
in Formula 1-2,
Ar is a phenyl substituted with one $R_2$,
$R_2$ is a $C_1$-$C_4$ alkyl group, a $C_1$-$C_4$ alkoxy group, an adamantane group, an acetyl group, or an acetylamino group,
in Formula 1-3,

Ar is a phenyl substituted with 1-3 $R_3$ or an unsubstituted naphthalene,
$R_3$ is a hydrogen or a $C_1$-$C_4$ alkyl group, wherein when more than one $R_3$ is present, all $R_3$ residues are identical, preferably $R_3$ is a $C_1$-$C_4$ alkyl group,
in Formula 1-4,
Ar is a phenyl unsubstituted or substituted with 1 to 3 $R_4$,
$R_4$ is a hydrogen, a phenyl group, a $C_1$-$C_4$ alkyl group, or a $C_1$-$C_4$ alkoxy group, wherein when more than one $R_4$ is present, all $R_4$ residues are identical,
in Formula 1-5,
Ar is a phenyl substituted with 1 to 3 $R_5$,
$R_5$ is a $C_1$-$C_4$ alkyl group, wherein when more than one $R_5$ is present, all $R_5$ residues are identical with each other.

**2.** The compound represented by formula 1 according to claim 1, comprising any one of the following compounds:

N-hydroxy-1-(phenylsulfonyl)piperidine-2-carboxamide,
1-([1,1'-biphenyl]-4-ylsulfonyl)-N-hydroxypiperidine-2-carboxamide,
1-((4-butoxyphenyl)sulfonyl)-N-hydroxypiperidine-2-carboxamide,
N-hydroxy-1-(mesitylsulfonyl)piperidine-2-carboxamide,
2-((4-(tert-butyl)phenyl)sulfonamido)-N-hydroxy-3-phenylpropanamide,
N-hydroxy-3-phenyl-2-((2,4,6-trimethylphenyl)sulfonamido)propanamide,
N-hydroxy-2-(naphthalene-2-sulfonamido)-3-phenylpropanamide,
2-([1,1'-biphenyl]-4-sulfonamido)-N-hydroxy-4-methylpentanamide,
2-((4-(tert-butyl)phenyl)sulfonamido)-N-hydroxy-4-methylpentanamide,
2-((4-butoxyphenyl)sulfonamido)-N-hydroxy-4-methylpentanamide,
N-hydroxy-4-methyl-2-((2,4,6-trimethylphenyl)sulfonamido)pentanamide,
2-((4-(tert-butyl)phenyl)sulfonamido)-N-hydroxy-3-methylbutanamide,
N-hydroxy-3-methyl-2-((2,4,6-trimethylphenyl)sulfonamido)butanamide,
1-((4-fluorophenyl)sulfonyl)-N-hydroxypiperidine-2-carboxamide,
1-((4-((3r,5r,7r)-adamantan-1-yl)phenyl)sulfonyl)-N-hydroxypiperidine-2-carboxamide,
N-hydroxy-1-((4-propylphenyl)sulfonyl)piperidine-2-carboxamide,
1-((4-acetamidophenyl)sulfonyl)-N-hydroxypiperidine-2-carboxamide,
1-((4-(tert-butyl)phenyl)sulfonyl)-N-hydroxypyrrolidine-2-carboxamide,
1-((4-butoxyphenyl)sulfonyl)-N-hydroxypyrrolidine-2-carboxamide,
1-((4-((3r,5r,7r)-adamantan-1-yl)phenyl)sulfonyl)-N-hydroxypyrrolidine-2-carboxamide,
N-hydroxy-1-((4-propylphenyl)sulfonyl)pyrrolidine-2-carboxamide,
1-((4-acetylphenyl)sulfonyl)-N-hydroxypyrrolidine-2-carboxamide,
N-hydroxy-1-((4-(1-(hydroxyimino)ethyl)phenyl)sulfonyl)piperidine-2-carboxamide,
N-hydroxy-4-methyl-2-(phenylsulfonamido)pentanamide, and
1-((4-acetamidophenyl)sulfonyl)-N-hydroxypyrrolidine-2-carboxamide.

**3.** The compound represented by formula 1 according to claim 1, comprising the any one of the following compounds:

N-hydroxy-1-(phenylsulfonyl)piperidine-2-carboxamide,
1-([1,1'-biphenyl]-4-ylsulfonyl)-N-hydroxypiperidine-2-carboxamide,
1-((4-butoxyphenyl)sulfonyl)-N-hydroxypiperidine-2-carboxamide,
N-hydroxy-1-(mesitylsulfonyl)piperidine-2-carboxamide,
2-((4-(tert-butyl)phenyl)sulfonamido)-N-hydroxy-3-phenylpropanamide,
N-hydroxy-3-phenyl-2-((2,4,6-trimethylphenyl)sulfonamido)propanamide,
N-hydroxy-2-(naphthalene-2-sulfonamido)-3-phenylpropanamide,
2-([1,1'-biphenyl]-4-sulfonamido)-N-hydroxy-4-methylpentanamide,
2-((4-(tert-butyl)phenyl)sulfonamido)-N-hydroxy-4-methylpentanamide,
2-((4-butoxyphenyl)sulfonamido)-N-hydroxy-4-methylpentanamide,
N-hydroxy-4-methyl-2-((2,4,6-trimethylphenyl)sulfonamido)pentanamide,
N-hydroxy-3-methyl-2-((2,4,6-trimethylphenyl)sulfonamido)butanamide,
1-((4-fluorophenyl)sulfonyl)-N-hydroxypiperidine-2-carboxamide,
N-hydroxy-1-((4-propylphenyl)sulfonyl)piperidine-2-carboxamide,
1-((4-acetamidophenyl)sulfonyl)-N-hydroxypiperidine-2-carboxamide,
1-((4-butoxyphenyl)sulfonyl)-N-hydroxypyrrolidine-2-carboxamide,
1-((4-((3r,5r,7r)-adamantan-1-yl)phenyl)sulfonyl)-N-hydroxypyrrolidine-2-carboxamide,

N-hydroxy-1-((4-propylphenyl)sulfonyl)pyrrolidine-2-carboxamide,
1-((4-acetylphenyl)sulfonyl)-N-hydroxypyrrolidine-2-carboxamide,
N-hydroxy-1-((4-(1-(hydroxyimino)ethyl)phenyl)sulfonyl)piperidine-2-carboxamide,
and
N-hydroxy-4-methyl-2-(phenylsulfonamido)pentanamide.

**4.** The compound represented by formula 1 according to claim 1, comprising the any one of the following compounds:

2-((4-(tert-butyl)phenyl)sulfonamido)-N-hydroxy-3-methylbutanamide,
1-((4-((3r,5r,7r)-adamantan-1-yl)phenyl)sulfonyl)-N-hydroxypiperidine-2-carboxamide,
1-((4-(tert-butyl)phenyl)sulfonyl)-N-hydroxypyrrolidine-2-carboxamide, and
1-((4-acetamidophenyl)sulfonyl)-N-hydroxypyrrolidine-2-carboxamide.

**5.** The compound represented by formula 1 according to claim 1, wherein the compound is derived from an amino acid.

**6.** A cosmetic composition comprising, at least one compound according to any one of claims 1 to 5.

**7.** The cosmetic composition according to claim 6, wherein a concentration of the at least one compound is 0.01 μM to 100 mM based on the total volume of the composition.

**8.** A food composition comprising, at least one compound according to any one of claims 1 to 5.

**9.** The food composition according to claim 8, wherein a concentration of the at least one compound is 0.01 μM to 100 mM based on the total volume of the composition.

**10.** A composition for inhibiting MMP-1 activity comprising, as an active ingredient, at least one compound according to any one of claims 1 to 5.

**11.** The composition for inhibiting the MMP-1 activity according to claim 10, wherein a concentration of the at least one is 0.01 μM to 100 mM based on the total volume of the composition.

**12.** A composition for inhibiting MMP-2 activity comprising, as an active ingredient, at least one compound according to any one of claims 1 to 5.

**13.** The composition for inhibiting the MMP-2 activity according to claim 12, wherein a content of the at least one compound is 0.01 μM to 100 mM based on the total volume of the composition.

**14.** A composition for inhibiting MMP-9 activity comprising, as an active ingredient, at least one compound according to any one of claims 1 to 5.

**15.** The composition for inhibiting the MMP-9 activity according to claim 14, wherein a content of the at least one compound is 0.01 μM to 100 mM based on the total volume of the composition.

**16.** A method for preparing a compound according to any one of claims 1 to 5, comprising the step of reacting an amino acid selected from pipecolinic acid, phenylalanine, leucine, valine and proline with any one selected from a sulfonic acid, a sulfonyl halide, a sulfanilic acid, and a sulfanilyl halide to obtain an intermediate.

**17.** The method according to claim 16, wherein the method further comprises the step of obtaining the compound represented by formula 1 by reacting the intermediate with a hydroxylamine in its free form or a hydroxylamine salt.

**18.** The method according to claim 16, wherein the sulfonyl halide is a sulfonyl fluoride, a sulfonyl chloride, a sulfonyl bromide, or a sulfonyl iodide, and the sulfanilyl halide is a sulfanylyl fluoride, a sulfanylyl chloride, a sulfanylyl bromide, or a sulfanylyl iodide.

**19.** The method according to claim 18, wherein the sulfonyl chloride is any one selected from a group of a benzenesulfonyl chloride, 4-biphenylsulfonyl chloride, 4-butoxybenzene-1-sulfonyl chloride, 2-mesitylenesulfonyl chloride, 4-tert-butylbenzenesulfonyl chloride, 2-naphthalenesulfonyl chloride, 4-fluorobenzenesulfonyl chloride, 4-(1-adamantyl)benzenesulfonyl chloride, 4-n-propylbenzenesulfonyl chloride, and 4-acetylbenzenesulfonyl chloride, and the sulfanylyl chloride is N-acetylsulfanylyl chloride.

**INTERNATIONAL SEARCH REPORT**

International application No.
**PCT/KR2024/004030**

**A. CLASSIFICATION OF SUBJECT MATTER**

**C07D 211/96**(2006.01)i; **C07C 311/21**(2006.01)i; **C07C 311/19**(2006.01)i; **C07D 207/48**(2006.01)i; **A61K 8/46**(2006.01)i; **A61K 8/49**(2006.01)i; **A61K 31/445**(2006.01)i; **A61K 31/40**(2006.01)i; **A61P 17/00**(2006.01)i

According to International Patent Classification (IPC) or to both national classification and IPC

**B. FIELDS SEARCHED**

Minimum documentation searched (classification system followed by classification symbols)

C07D 211/96(2006.01); A61K 31/18(2006.01); C07C 311/19(2006.01); C07C 311/46(2006.01); C07D 213/55(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, Caplus) & keywords: 설포닐화 아미노산 하이드록사메이트(sulfonylated amino acid hydroxamate), 기질금속단백질분해효소(matrix metalloproteinase, MMP), 화장료 조성물(cosmetic composition)

**C. DOCUMENTS CONSIDERED TO BE RELEVANT**

| Category* | Citation of document, with indication, where appropriate, of the relevant passages | Relevant to claim No. |
|---|---|---|
| X<br>Y | SCOZZAFAVA, A. et al. Carbonic anhydrase and matrix metalloproteinase inhibitors: sulfonylated amino acid hydroxamates with MMP inhibitory properties act as efficient inhibitors of CA isozymes I, II, and IV, and N-hydroxysulfonamides inhibit both these zinc enzymes. J. Med. Chem. 2000, vol. 43, pp. 3677-3687.<br>See abstract; table 1; and formula 1. | 1-5,10-19<br>6-9 |
| Y | KR 10-2008-0104347 A (NOVARTIS AG) 02 December 2008 (2008-12-02)<br>See abstract; and paragraphs [0178], [0187] and [0190]. | 6-9 |
| X | ALMSTEAD, N. G. et al. Design, synthesis, and biological evaluation of potent thiazine- and thiazepine-based matrix metalloproteinase inhibitors. J. Med. Chem. 1999, vol. 42, pp. 4547-4562.<br>See abstract; and table 1 (compounds 37 and 39). | 1,10 |
| X | LENCI, E. et al. Identification of highly potent and selective MMP2 inhibitors addressing the S1′ subsite with D-proline-based compounds. Bioorganic & medicinal chemistry. 2019, vol. 27, pp. 1891-1902.<br>See abstract; and table 1 (compound 5). | 1,12,14 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **25 June 2024** | **26 June 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.
**PCT/KR2024/004030**

**C. DOCUMENTS CONSIDERED TO BE RELEVANT**

| Category* | Citation of document, with indication, where appropriate, of the relevant passages | Relevant to claim No. |
|---|---|---|
| X | JP 11-246527 A (SHIONOGI & CO., LTD.) 14 September 1999 (1999-09-14) See page 10 (compound 85). | 1 |

Form PCT/ISA/210 (second sheet) (July 2022)

**INTERNATIONAL SEARCH REPORT**
**Information on patent family members**

International application No.
**PCT/KR2024/004030**

| Patent document cited in search report | Publication date (day/month/year) | Patent family member(s) | Publication date (day/month/year) |
|---|---|---|---|
| KR 10-2008-0104347 A | 02 December 2008 | CN 101410370 A | 15 April 2009 |
| | | CN 101410370 B | 12 June 2013 |
| | | CN 103420877 A | 04 December 2013 |
| | | CN 103420877 B | 12 August 2015 |
| | | EP 2001838 A2 | 17 December 2008 |
| | | EP 2001838 B1 | 12 March 2014 |
| | | EP 2004500 A2 | 24 December 2008 |
| | | EP 2597083 A2 | 29 May 2013 |
| | | EP 2597083 A3 | 17 September 2014 |
| | | JP 2009-535299 A | 01 October 2009 |
| | | JP 5160535 B2 | 13 March 2013 |
| | | US 2009-0318511 A1 | 24 December 2009 |
| | | US 2012-0225882 A1 | 06 September 2012 |
| | | US 8232427 B2 | 31 July 2012 |
| | | US 8314148 B2 | 20 November 2012 |
| | | WO 2007-112376 A2 | 04 October 2007 |
| | | WO 2007-112376 A3 | 04 September 2008 |
| | | WO 2007-117981 A2 | 18 October 2007 |
| | | WO 2007-117981 A3 | 13 December 2007 |
| JP 11-246527 A | 14 September 1999 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020230042219
- KR 1020240041934